# EUROPEAN PATENT SPECIFICATION

(11) **EP 3 350 316 B1**
(45) Date of publication and mention of the grant of the patent: **08.05.2024**
(21) Application number: 16775091.8
(22) Date of filing: 14.09.2016
(51) Int. Cl.: A61K 35/17, C12N 5/0783, A61P 35/00

(54) **IMPROVED METHOD FOR EX VIVO EXPANSION CD34+HSPCs INTO NK CELLS USING AN ARYL HYDROCARBON RECEPTOR ANTAGONIST**
VERBESSERTES VERFAHREN ZUR EX-VIVO-EXPANSION VON CD34+HSPCs IN NK-ZELLEN UNTER VERWENDUNG EINES ARYL-KOHLENWASSERSTOFF-REZEPTORANTOGANISTEN
PROCÉDÉ AMÉLIORÉ POUR L'EX VIVO EXPANSION DE CD34+HSPCs DANS DES CELLULES NK UTILISANT UN ANTAGONISTE DU RÉCEPTEUR DES HYDROCARBURES DU GROUPE ARYLE

(30) Priority: 15.09.2015 EP 15185324
(43) Date of publication of application: 25.07.2018
(73) Proprietor: Stichting Radboud universitair medisch centrum, 6525 GA Nijmegen (NL)
(72) Inventor: DOLSTRA, Harmen, 6605 XL Wijchen (NL)
(74) Representative: Nederlandsch Octrooibureau
(86) International application number: PCT/EP2016/071660
(87) International publication number: WO 2017/046142

(56) References cited:
- WO-A1-2013/119118
- WO-A1-2016/109661
- PAOLO AMBROSINI ET AL: "IL-1[beta] inhibits ILC3 while favoring NK-cell maturation of umbilical cord blood CD34 + precursors", EUROPEAN JOURNAL OF IMMUNOLOGY, vol. 45, no. 7, 12 May 2015 (2015-05-12), pages 2061-2071, XP055222638, DE ISSN: 0014-2980, DOI: 10.1002/eji.201445326
- JAN SPANHOLTZ ET AL: "High log-scale expansion of functional human natural killer cells from umbilical cord blood CD34-positive cells for adoptive cancer immunotherapy", PLOS ONE, PUBLIC LIBRARY OF SCIENCE, US, vol. 5, no. 2, 15 February 2010 (2010-02-15), pages e9221-1, XP002665360, ISSN: 1932-6203, DOI: 10.1371/JOURNAL.PONE.0009221
- TIFFANY HUGHES ET AL: "The Transcription Factor AHR Prevents the Differentiation of a Stage 3 Innate Lymphoid Cell Subset to Natural Killer Cells", CELL REPORTS, vol. 8, no. 1, 1 July 2014 (2014-07-01), pages 150-162, XP055222519, US ISSN: 2211-1247, DOI: 10.1016/j.celrep.2014.05.042
- ANTHONY E. BOITANO ET AL: "Aryl Hydrocarbon Receptor Antagonists Promote the Expansion of Human Hematopoietic Stem Cells", SCIENCE, vol. 329, no. 5997, 5 August 2010 (2010-08-05), pages 1345-1348, XP055558705, US ISSN: 0036-8075, DOI: 10.1126/science.1191536
- ROEVEN ET AL.: "The Aryl Hydrocarbon Receptor Antagonist StemRegenin 1 stimulates expression of NK cell related transcription factors, ...", BLOOD, vol. 124, no. 21, 6 December 2014 (2014-12-06), page 3833, XP009522110,
- MIEKE W.H. ROEVEN ET AL: "The Aryl Hydrocarbon Receptor Antagonist StemRegenin1 Improves In Vitro Generation of Highly Functional Natural Killer Cells from CD34 + Hematopoietic Stem and Progenitor Cells", STEM CELLS AND DEVELOPMENT, vol. 24, no. 24, 28 September 2015 (2015-09-28), pages 2886-2898, XP055561683, US ISSN: 1547-3287, DOI: 10.1089/scd.2014.0597

## Description

### FIELD OF THE INVENTION

The present invention relates to the field of medicine, specifically the field of treatment of cancer. More specifically, the invention relates to a method for the *ex vivo* production of a population of highly functional NK cells from CD34-positive cells, to a population of highly functional NK cells obtained and to the use of such population of highly functional NK cells for adoptive cell therapy.

### BACKGROUND OF THE INVENTION

Natural killer cells (NK cells) are CD3-negative CD56-positive lymphocytes, which are part of the innate immune system and play an important role in the defense against virus-infected and transformed cells. NK cell activation and subsequent killing of target cells is regulated by a balance in their expression levels of inhibitory receptors, including the killer-immunoglobulin like receptors (KIRs) and CD94/NKG2A heterodimer, versus activating receptors, such as DNAX accessory molecule-1 (DNAM-1), natural cytotoxicity receptors (NCRs) and NKG2D. In homeostasis, NK cells are inhibited by their inhibitory receptors recognizing self human leukocyte antigen (HLA) class I molecules and/or HLA-E molecules presenting conserved HLA class I leader sequences. However, an NK cell-mediated anti-tumor effect can be induced by up-regulation of activating ligands or down-regulation of HLA class I molecules on tumor cells. In addition, in the setting of haploidentical allogeneic stem cell transplantation (allo-SCT), donor NK cells may lack expression of inhibitory KIRs for recipient HLA class I molecules and hence be activated. This phenomenon is called missing-self recognition and can contribute to the curative graft-versus-tumor (GVT) effect [1].

Because of their ability to kill tumor cells, NK cells are considered potent effectors for adoptive immunotherapy against cancer. So far, promising results have been obtained by infusion of haploidentical NK cells after immunosuppressive chemotherapy in adult and childhood acute myeloid leukemia (AML) [2-4]. However, a limitation in these studies is the relatively low NK cell numbers that can be enriched from aphaeresis products for multiple infusions. Furthermore, contaminating alloreactive T cells risk the induction of graft-versus-host disease (GVHD), especially when IL-2 of IL-15 are co-administrated to boost NK cell survival and expansion. In order to generate high numbers of allogeneic NK cells completely devoid of T cell contamination, a Good Manufacturing Practice (GMP)-compliant, cytokine-based *ex vivo* culture protocol has been developed [5,6]. Using this procedure, CD34+ hematopoietic stem and progenitor cells (HSPCs) isolated from umbilical cord blood (UCB) can be expanded over 2000-fold in large-scale bioreactors into a mixture of immature and mature NK cells with a purity >80%. Pre-clinical studies conducted in NOD/SCID-IL2Rγnull (NSG) mice demonstrated that these HSPC-NK cells have BM homing capacity, display IL-15-driven *in vivo* expansion, further mature *in vivo* by gaining CD16 and KIR expression, and effectively prolong survival of leukemia-bearing mice [7]. Currently, administration of this HSPC-NK cell product following immunosuppressive chemotherapy is being investigated in a phase I clinical trial in older AML patients who are not eligible for allo-SCT (see www.trialregister.nl and search for 2818).

In HLA-matched non-myeloablative and T cell-depleted allo-SCT, early NK cell repopulation has been associated with decreased relapse rates, without increasing GVHD incidence [8,9]. Moreover, high NK cell numbers in stem cell grafts have been associated with a decreased incidence of GVHD [10]. In addition, transplants from donors with KIR-B haplotypes, containing several activating KIRs, led to lower rates of relapse and improved survival [11-13]. For these reasons, it would be highly valuable to exploit HSPC-NK cell products for adoptive immunotherapy after allo-SCT. Since NK cells of donor origin will not be rejected, multiple NK cell infusions without the need for immunosuppressive chemotherapy to prevent rejection, could be administered after allo-SCT. Consequently, these cells may potentially induce long-term GVT effects. However, to obtain large numbers of NK cells from donor origin, peripheral blood (PB) or bone marrow (BM)-derived CD34-positive HSPCs, which have a lower expansion potential compared to UCB-derived CD34-positive HSPCs, should be expanded and differentiated into NK cells.

As said, a protocol for obtaining high numbers of NK cells has been reported [5,6]. The protocol is however based on CD34-positive cells from umbilical cord blood, which cells have a higher expansion potential than peripheral blood (PB) or bone marrow (BM)-derived CD34-positive HSPCs. In addition, said protocol requires the use of heparin from animal origin which has the disadvantage that it may be contaminated with pathogens and its composition may not be constant over time. Moreover, the protocol requires a low dose cytokine cocktail throughout the procedure which renders the protocol complicated and expensive. Furthermore, GMP-compliant productions using this heparin-based protocol has shown some inconsistencies regarding NK purity of more than 80% (range 40-85%, n=12, Dolstra et al. manuscript submitted).

Accordingly, there is a need for more robust and consistent *ex vivo* expansion and differentiation of CD34-positive HSPCs into NK cells. Such method should meet the requirements of resulting in high-fold expansion in large-scale bioreactors, high purity of the NK cells and high functionality of the NK cells. Effective HSPC-NK products can be exploited for adoptive cell therapy in allogeneic stem cell transplant as well as non-transplant setting against hematological and solid malignancies.

### SUMMARY OF THE INVENTION

The invention is set out in the appended set of claims.

### DETAILED DESCRIPTION OF THE INVENTION

Notably, it has now been demonstrated that peripheral blood (PB) or bone marrow (BM)-derived CD34-positive HSPCs, next to UCB-derived CD34-positive HSPCs, can be expanded and differentiated into a population of highly functional NK cells. Accordingly, in a first aspect, there is disclosed a method, preferably a consistent method, for the *ex vivo* production of a population of highly functional NK cells from CD34-positive cells comprising culturing CD34-positive cells in an expansion medium and subsequently culturing the expanded CD34-positive cells in a differentiation medium, wherein the expansion medium comprises an aryl hydrocarbon receptor antagonist, wherein the CD34-positive cells are preferably peripheral blood, bone marrow-derived or umbilical cord blood CD34-positive cells. Said method is herein referred to as a method as disclosed herein. The CD34-positive cells are preferably HSPCs and may be obtained or have been obtained from any source. Preferably, the CD34-positive cells are obtained or have been obtained from a human subject. A preferred source of the CD34-positive cells is blood, bone marrow or other post-embryonic tissue such as adult fat tissue (mesenchymal stem cells) and umbilical cord blood. The CD34-positive cells may also be obtained by reprogramming tissue specific cells such as skin stem cells or fibroblasts to produce HSPCs from induced pluripotent stem cells. The CD34-positive cells may or may not be obtained from embryonic stem cell lines. Particularly preferred are peripheral blood (PB), bone marrow (BM)-derived or umbilical cord blood (UCB) CD34-positive HSPCs.

CD34-positive cells may be isolated or may have been isolated using any means known in the art. After collection of a sample (e.g. a bone marrow sample, peripheral blood sample or umbilical cord blood sample), mononuclear cells may be isolated using density gradient centrifugation. Peripheral blood cells may be obtained or may have been obtained by aphaeresis material from donors such as stem cell donors that may have been treated with G-SCF (granulocyte- colony stimulating factor). CD34-positive cells are preferably isolated from a population that may have been enriched for mononuclear cells using affinity purification such as using an anti-CD34 antibody, preferably using anti-CD34 immunomagnetic beads. A preferred method for the isolation of CD34-positive cells is a method as described in the examples herein. The isolated CD34-positive cells may be used fresh, i.e. immediately of shortly after isolation, or may be stored until further use.

The expansion media and differentiation media described herein are collectively referred to as an expansion medium as disclosed herein and a differentiation medium as disclosed herein. An expansion medium as disclosed herein and a differentiation medium as disclosed herein comprise a basic medium supplemented with further components as defined herein. The basic medium may be any medium suitable for propagation and/or maintenance of CD34-positive HSPCs and/or for propagation and/or maintenance of CD56-positive cells. The basic medium is preferably a GMP (Good Manufacturing Practice) medium. The basic medium is preferably Cellgro GMP DC medium (Cellgenix, Freiburg, Germany) or NK MACS medium (Miltenyi, Bergisch Gladbach, Germany) supplemented with 2-10% human serum (such as human serum obtainable from Sanquin Bloodbank, Nijmegen, The Netherlands). A preferred basic medium is one described in the examples herein.

*Ex vivo* production is to be construed that it does not involve any invasive manipulation of the human or animal body since it involves a method performed on a sample that has already been provided. A method as disclosed herein may be an *in vitro* method.

A population of cells is to be construed as at least 1E+3 viable NK cells. Preferably, a population of cells comprises at least 1E+4, more preferably 1E+5, 1E+6, 1E+7, 1E+8, or most preferably 1E+9 viable NK cells.

Preferably, in a method as disclosed herein the CD34-positive cells are cultured at an initial concentration of at least 1E+5 cells/ml, preferably between 1E+5 and 5E+5 cells/ml in a plate, flask or bag. Preferably, after about three days of culture, the cells are transferred to a new plate, flask or bag to deplete for stromal cells. Preferably, the cultures are refreshed with about at least 20% fresh medium every two to three days. Cell cultures are typically maintained under conditions conducive to the propagation and/or maintenance of the cells. Preferably, the cell cultures are maintained at around 37°C in 75-95% humidity in 4.5-5.5% CO₂. Preferably, the total culture time-frame for expansion and differentiation of CD34-positive cells into highly functional NK cells is about five weeks to about seven weeks, preferably about five to about six weeks and most preferably about five weeks.

In a method as disclosed herein, an expansion medium does not comprise a glycosaminoglycan and does not comprise G-CSF (granulocyte- colony-stimulating factor), GM-CSF (granulocyte-macrophage-colony-stimulating factor) or IL-6 (interleukin-6). An expansion medium as disclosed herein does not comprise a glycosaminoglycan, G-CSF, GM-CSF or IL-6.

In a method as disclosed herein, a differentiation medium does not comprise a glycosaminoglycan, G-CSF, GM-CSF or IL-6. A differentiation medium as disclosed herein does not comprise a glycosaminoglycan, G-CSF, GM-CSF or IL-6.

Preferably, such glycosaminoglycan is a heparin such as a low molecular weight heparin (LMWH). A LMWH typically is a heparin or heparin salt having an average molecular weight of between about 2000-10000 Dalton, preferably between 5000 and 8000 Dalton and more preferably about 8000 Dalton. Such heparin may be acetylated, desulphated and/or phosphorylated.

In a method according to the invention culturing CD34-positive cells in an expansion medium and subsequently culturing the expanded CD34-positive cells in a differentiation medium comprises:
- culturing in an expansion medium comprising IL-7, SCF, TPO, Flt3L and an aryl hydrocarbon receptor antagonist,
- further culturing in a first differentiation medium comprising IL-7, SCF, Flt3L, IL-15 and an aryl hydrocarbon receptor antagonist,
- culturing in a second differentiation medium comprising IL-7, SCF and IL-15.

In a method as disclosed herein, in the expansion medium, the concentration of IL-7 is preferably in the range of about 10 and 150ng/ml, more preferably in the range of about 20 to 120ng/ml, more preferably about 25ng/ml.

In a method as disclosed herein, in the expansion medium, the concentration of SCF is preferably in the range of about 10 and 150ng/ml, more preferably in the range of about 20 to120/ml, more preferably about 25ng/ml.

In a method as disclosed herein, in the expansion medium, the concentration of TPO is preferably in the range of about 10 and 150ng/ml, more preferably in the range of about 20 to 120ng/ml, more preferably about 25ng/ml.

In a method as disclosed herein, in the expansion medium, the concentration of Flt3L is preferably in the range of about 10 and 150ng/ml, more preferably in the range of about 20 to 120ng/ml, more preferably about 25ng/ml.

In a method as disclosed herein, in the expansion medium, the concentration of aryl hydrocarbon receptor antagonist is preferably in the range of about 0.5 and 5µm, more preferably in the range of about 1 to 5µm, more preferably about 2µm.

In a method as disclosed herein, in the first differentiation medium, the concentration of IL-7 is preferably in the range of about 10 and 40ng/ml, more preferably in the range of about 20 to 30ng/ml, more preferably about 25ng/ml.

In a method as disclosed herein, in the first differentiation medium, the concentration of SCF is preferably in the range of about 10 and 40ng/ml, more preferably in the range of about 20 to 30ng/ml, more preferably about 25ng/ml.

In a method as disclosed herein, in the first differentiation medium, the concentration of IL-15 is preferably in the range of about 10 and 100ng/ml, more preferably in the range of about 25 to 75ng/ml, more preferably about 50ng/ml.

Preferably, in a method as disclosed herein the first differentiation medium and/or the second differentiation medium further comprises IL-2 (interleukin-2) or IL-12 (interleukin-12). Preferably, the first differentiation medium further comprises IL-2 or IL-12, preferably IL-12. Preferably, the second differentiation medium further comprises IL-2 or IL-12, preferably IL-12. Preferably, the first differentiation medium and the second differentiation medium further comprises IL-2 or IL-12, preferably IL-12. Preferably, the concentration of IL-2 is in the range of about 500 to 2000U/ml, more preferably in the range of about 750 to 1500U/ml, more preferably about 1000U/ml. Preferably, the concentration of IL-12 is in the range of about 0.05 to 0.4ng/ml, more preferably in the range of about 0.1 to 0.3 ng/ml, more preferably about 0.2ng/ml. Preferably, in a method as disclosed herein, the expansion medium comprises about 25ng/ml IL-7, about 25ng/ml CSF, about 25ng/ml TPO, about 25ng/ml Flt3L and about 2µm aryl hydrocarbon receptor antagonist, preferably SR1.

Preferably, in a method as disclosed herein, the first differentiation medium comprises about 25ng/ml IL-7, about 25ng/ml CSF, about 25ng/ml Flt3L, about 50ng/ml IL-15 and about 2µm aryl hydrocarbon receptor antagonist, preferably SR1.

Preferably, in a method as disclosed herein, the second differentiation medium comprises about 20ng/ml IL-7, about 20ng/ml CSF, about 250ng/ml IL-15, about 1000U/ml IL-2 or about 0.2ng/ml IL-12 and about 2µm aryl hydrocarbon receptor antagonist, preferably SR1. More preferably, the second differentiation medium comprises about 20ng/ml IL-7, about 20ng/ml CSF, about 250ng/ml IL-15 and about 1000U/ml IL-2 or about 0.2ng/ml IL-12.

In an embodiment:
- the expansion medium comprises about 25ng/ml IL-7, about 25ng/ml CSF, about 25ng/ml TPO, about 25ng/ml Flt3L and about 2µm aryl hydrocarbon receptor antagonist, preferably SR1;
- the first differentiation medium comprises about 25ng/ml IL-7, about 25ng/ml CSF, about 25ng/ml Flt3L, about 50ng/ml IL-15 and about 2µm aryl hydrocarbon receptor antagonist, preferably SR1;
- the second differentiation medium comprises about 20ng/ml IL-7, about 20ng/ml CSF, about 250ng/ml IL-15, about 1000U/ml IL-2 or about 0.2ng/ml IL-12 and about 2µm aryl hydrocarbon receptor antagonist, preferably SR1.

In an embodiment:
- the expansion medium comprises about 25ng/ml IL-7, about 25ng/ml CSF, about 25ng/ml TPO, about 25ng/ml Flt3L and about 2µm aryl hydrocarbon receptor antagonist, preferably SR1;
- the first differentiation medium comprises about 25ng/ml IL-7, about 25ng/ml CSF, about 25ng/ml Flt3L, about 50ng/ml IL-15 and about 2µm aryl hydrocarbon receptor antagonist, preferably SR1;
- the second differentiation medium comprises about 20ng/ml IL-7, about 20ng/ml CSF, about 250ng/ml IL-15 and about 1000U/ml IL-2 or about 0.2ng/ml IL-12.

In an embodiment:
- the expansion medium comprises about 25ng/ml IL-7, about 25ng/ml CSF, about 25ng/ml TPO, about 25ng/ml Flt3L and about 2µm aryl hydrocarbon receptor antagonist, preferably SR1;
- the first differentiation medium comprises about 25ng/ml IL-7, about 25ng/ml CSF, about 25ng/ml Flt3L, about 50ng/ml IL-15 and about 2µm aryl hydrocarbon receptor antagonist, preferably SR1;
- the second differentiation medium comprises about 20ng/ml IL-7, about 20ng/ml CSF, about 250ng/ml IL-15, about 0.2ng/ml IL-12 and about 2µm aryl hydrocarbon receptor antagonist, preferably SR1.

In an embodiment:
- the expansion medium comprises about 25ng/ml IL-7, about 25ng/ml CSF, about 25ng/ml TPO, about 25ng/ml Flt3L and about 2µm aryl hydrocarbon receptor antagonist, preferably SR1;
- the first differentiation medium comprises about 25ng/ml IL-7, about 25ng/ml CSF, about 25ng/ml Flt3L, about 50ng/ml IL-15 and about 2µm aryl hydrocarbon receptor antagonist, preferably SR1;
- the second differentiation medium comprises about 20ng/ml IL-7, about 20ng/ml CSF, about 250ng/ml IL-15 and about 0.2ng/ml IL-12.

In an embodiment, the recipes of the media are as depicted in the examples herein.

In a method according to the invention, the aryl hydrocarbon receptor antagonist may be any the aryl hydrocarbon receptor antagonist that enhances expansion of CD34-positive HSPCs and induces expression of NK cell associated transcription factors promoting NK cell differentiation; it may be an antagonist that interferes with the aryl hydrocarbon receptor itself but is may also be an agent that reduces expression, transcription and/or translation of the aryl hydrocarbon receptor. A preferred aryl hydrocarbon receptor antagonist is one selected from the group consisting of SR1 (StemRegenin 1, WO2010/059401; Boitano et al. 2010), CH-223191 (Kim et al. 2006, Hughes et al. 2014), GNF351 (Smith et al. 2011), 6,2',4'trimethoxyflavone (Zhao et al. 2010) and CB7993113 (Parks et al. 2014). A more preferred aryl hydrocarbon receptor in the embodiments of the invention is SR1.

Preferably, in a method as disclosed herein, culturing in the expansion medium is performed for about 7 to 10 days. Preferably, culturing in the first differentiation medium is performed from about day 7 to 10 to about day 19 to 21. Preferably, culturing in the second differentiation medium is performed from about day 19 to 21 for at least about 10 days, more preferably for at least about 14 to about 21 days or for at least about 21 days.

Preferably, in a method as disclosed herein, culturing in the expansion medium is performed for about 7 to 10 days, culturing in the first differentiation medium is performed from about day 7 to 10 to about day 19 to 21 and culturing in the second differentiation medium is performed from about day 19 to 21 for at least about 10 days, more preferably for at least about 14 days, more preferably for at least about 21 days. More preferably, in a method as disclosed herein, culturing in the expansion medium is performed for about 9 to 10 days, culturing in the first differentiation medium is performed from about day 9 to 10 to about day 19 to 21 and culturing in the second differentiation medium is performed from about day 19 to 21 for at least about 10 days, more preferably for at least about 14 days, more preferably for at least about 21.

Preferably, in a method as disclosed herein at least about 1E+8, more preferably at least about 1E+9 highly functional NK cells are produced from a single donor or cord blood unit.

Preferably, in a method as disclosed herein the amount of non-NK cells is 20% or less in the produced population of highly functional NK cells. Preferably, the amount of highly functional NK cells is at least about 70%, more preferably at least about 75%, 76%, 77%, 78%, 79%, 80%, 81%, 82%, 83%, 84%, 85%, 86%, 87%, 88%, 89%, 90%, 91%, 92%, 93%, 94%, or at most about 95%.

Preferably, in a method as disclosed herein the amount of CD3-positive T cells is at most about 0.5%, more preferably at most about 0.4%, 0.3%, 0.2%, 0.1%, or at most about 0.05% in the produced population of highly functional NK cells.

Preferably, in a method as disclosed herein the mean overall expansion is at least about two-fold higher, more preferably at least about three-fold higher than when no aryl hydrocarbon receptor antagonist is used. Preferably, the mean overall expansion is at least 50-fold, 60-fold, 70-fold, 80-fold, 90-fold, 100-fold, 110-fold, 120-fold, 130-fold, 140-fold, 150-fold, 160-fold, 170-fold, 180-fold, 190-fold, 200-fold, 210-fold, 220-fold, 230-fold, 240-fold, 250-fold, 260-fold, 270-fold, 280-fold, 290-fold, 300-fold, 400-fold, 500-fold, 600-fold, 700-fold, 800-fold, 900-fold, 1000-fold, 1100-fold, 1200-fold, 1300-fold, 1400-fold, 1500-fold, 1600-fold, 1700-fold, 1800-fold, 1900-fold or more preferably at least 2000-fold. Preferably, the mean overall expansion for peripheral blood (PB) or bone marrow (BM)-derived CD34-positive HSPCs is at least 50-fold, 60-fold, 70-fold, 80-fold, 90-fold, 100-fold, 110-fold, 120-fold, 130-fold, 140-fold, 150-fold, 160-fold, 170-fold, 180-fold, 190-fold, 200-fold, 210-fold, 220-fold, 230-fold, 240-fold, 250-fold, 260-fold, 270-fold, 280-fold, 290-fold or more preferably at least 300-fold. Preferably, the mean overall expansion for umbilical cord blood (UCB) CD34-positive HSPCs is at least 300-fold, 400-fold, 500-fold, 600-fold, 700-fold, 800-fold, 900-fold, 1000-fold, 1100-fold, 1200-fold, 1300-fold, 1400-fold, 1500-fold, 1600-fold, 1700-fold, 1800-fold, 1900-fold or more preferably at least 2000-fold.

In a second aspect, there is disclosed a population of highly functional NK cells obtainable by a method as disclosed herein, preferably a method according to the first aspect as disclosed herein, preferably a method as depicted in the examples herein. Such population of highly functional NK cells is herein referred to as a population of highly functional NK cells as disclosed herein. A population of highly functional NK cells as disclosed herein is preferably an *ex vivo* or an *in vitro* population.

A population of highly functional NK cells as disclosed herein comprises at least 1E+9 highly functional NK cells from a single donor or cord blood unit.

Preferably, in a population of highly functional NK cells as disclosed herein the amount of non-NK cells is 20% or less. Preferably, the amount of highly functional NK cells is at least about 70%, more preferably at least about 75%, 76%, 77%, 78%, 79%, 80%, 81%, 82%, 83%, 84%, 85%, 86%, 87%, 88%, 89%, 90%, 91%, 92%, 93%, 94%, or at most about 95%.

In a population of highly functional NK cells as disclosed herein the amount of CD3-positive cells is at most about 0.1%, or at most about 0.05%.

In the embodiments of the invention, a population of highly functional NK cells is defined as a population wherein at least 10% of the CD56-positive cells is capable of secreting IFN-gamma upon stimulation with K562 target cells.

Preferably, a population of highly functional NK cells as disclosed herein is defined as a population wherein at least 10%, 20%, 30%, 40%, 50%, 60%, 70%, 80%, 81%, 82%, 83%, 84%, 85%, 86%, 87%, 88%, 89%, 90%, 91%, 92%, 93%, 94%, 95%, 96%, 97%, 98%, 99% or more preferable 100% of the CD56+ NK cells further express at least the combination of NKG2D, DNAM1, NKp46, TRAIL and CXCR3.

Preferably, a population of highly functional NK cells as disclosed herein is defined as a population wherein at least 10%, 20%, 30%, 40%, 50%, 60%, 70%, 80%, 81%, 82%, 83%, 84%, 85%, 86%, 87%, 88%, 89%, 90%, 91%, 92%, 93%, 94%, 95%, 96%, 97%, 98%, 99% or more preferable 100% of the CD56+ NK cells further express at least the combination of NKG2D, DNAM1, NKp30, NKp44, NKp46, TRAIL and CXCR3. Preferably, a population of highly functional NK cells as disclosed herein is defined as a population wherein at least 10%, 20%, 30%, 40%, 50%, 60%, 70%, 80%, 81%, 82%, 83%, 84%, 85%, 86%, 87%, 88%, 89%, 90%, 91%, 92%, 93%, 94%, 95%, 96%, 97%, 98%, 99% or more preferable 100% of the CD56+ NK cells further express at least the combination of NKG2D, DNAM1, NKp46, TRAIL, CXCR3 and perforin, granzymeB. Preferably, a population of highly functional NK cells as disclosed herein is defined as a population wherein at least 10%, 20%, 30%, 40%, 50%, 60%, 70%, 80%, 81%, 82%, 83%, 84%, 85%, 86%, 87%, 88%, 89%, 90%, 91%, 92%, 93%, 94%, 95%, 96%, 97%, 98%, 99% or more preferable 100% of the CD56+ NK cells further express at least the combination of NKG2D, DNAM1, NKp30, NKp44, NKp46, TRAIL, CXCR3, perforin, granzymeB and are capable of secreting IFN-gamma upon stimulation with K562 target cells.

More preferably, a population of highly functional NK cells as disclosed herein is defined as a population wherein at least 10%, 20%, 30%, 40%, 50%, 60%, 70%, 80%, 81%, 82%, 83%, 84%, 85%, 86%, 87%, 88%, 89%, 90%, 91%, 92%, 93%, 94%, 95%, 96%, 97%, 98%, 99% or more preferable 100% of the CD56+ NK cells further express at least the combination of NKG2A, NKG2D, DNAM1, NKp30, NKp44, NKp46, TRAIL, CD62L, CXCR3, perforin, granzyme B and are capable of secreting IFN-gamma upon stimulation with K562 target cells.

Further disclosed is a population of NK cells, wherein in the population, at least 80% of the NK cells are highly functional NK cells, more preferably wherein the highly functional NK cells are CD56-positive, Perforin-positive and EOMES-positive NK cells. Further disclosed is a therapeutic product comprising a population of NK cells as disclosed herein

In a third aspect, there is disclosed the medical use of a population of highly functional NK cells as disclosed herein, for the medical use of a population of highly functional NK cells obtainable by a method as disclosed herein and for the medical use of a therapeutic product comprising a population of NK cells as disclosed herein. In such medical use, a population of highly functional NK cells as disclosed herein or a population of highly functional NK cells obtainable by a method as disclosed herein, or a therapeutic product comprising a population of NK cells as disclosed herein can be transplanted into a patient by means known to the person skilled in the art.

Accordingly, the invention provides for a population of highly functional NK cells as disclosed herein or a population of highly functional NK cells obtainable by a method as disclosed herein or a therapeutic product comprising a population of NK cells as disclosed herein for use as a medicament. Preferably, the use as a medicament is the treatment of a cancer, a viral disease, a fungal disease, or a solid transplant rejection, or an autoimmune disease and a loss of pregnancy. Preferably, the use as a medicament is the treatment of a cancer. Accordingly, there is provided a population of cells as disclosed herein or a population of highly functional NK cells obtainable by a method as disclosed herein or a therapeutic product comprising a population of NK cells as disclosed herein for use in the treatment of cancer. Said cancer may be any type of cancer suitable for treatment by NK cells, such as but not limited to a skin cancer, breast cancer, lung cancer, ovarian cancer, fallopian tube cancer, colorectal cancer, head and neck cancer, prostate cancer, bladder cancer, liver cancer, pancreatic cancer, stomach cancer, esophagus cancer, brain cancer and melanoma. Preferably, said cancer is a cancer of hematopoietic origin like leukemia such as acute myelogenous leukemia (AML), acute lymphoblastic leukemia (ALL), chronic myelogenous leukemia (CML), chronic lymphocytic leukemia (CLL), and lymphomas such as Hodgkin's and Non-Hodgkin's Lymphomas (HL and NHL) and their subtypes or multiple myeloma (MM).

The medical use of a population of highly functional NK cells as disclosed herein or a therapeutic product comprising a population of NK cells as disclosed herein may further comprise another type of treatment such as treatment of a cancer with a therapeutic antibody specific for an antigen present on cells of said cancer in order to induce antibody-dependent cell-mediated cytotoxicity (ADDC) of the transferred NK cells. Preferably, such antibody is a therapeutic monoclonal antibody such as, but not limited to, Rituximab (anti-CD20), Ofatumumab (anti-CD20), Tositumomab (anti-CD20), Ibritumomab (anti-CD20), Brentuximab (anti-CD30), Dacetuzumab (anti- CD40), Trastuzumab (anti-Her2), Alemtuzumab (anti-CD52), Cetuximab (anti-EGFR), Panitumumab (anti-EGFR), Gemtuzumab (anti-CD33), Elotuzumab (anti-SLAM7), Catumaxomab (anti-EpCam), G250 (anti-CAIX). The medical use of a population of highly functional NK cells as disclosed herein may further comprise treatment with antibodies that influence migration or function of transferred NK cells such as, but not limited to, Bevacizumab (anti-VEGF), Tremelimumab (anti-CTLA-4), Ipilimumab (anti-CTLA4), Pidilizumab (anti-PD-l), Nivolumab (anti-PD-1), Pembrolizumab (anti-PD1), Lirilumab (anti-KIR). The medical use of a population of highly functional NK cells as disclosed herein may further comprise treatment with epigenetic modifiers such as, but not limited to, DNA methylation inhibitors such as, but not limited to, Decitabine and Azacitadine, and histon deacetylase inhibitors such as, but not limited to, Valproic acid, Vorinostat and Panobinostat.

In an embodiment, there is provided a population of highly functional NK cells as disclosed herein or a population of highly functional NK cells obtainable by a method as disclosed herein or a therapeutic product comprising a population of NK cells as disclosed herein for use in the treatment in the treatment of cancer wherein treatment of the cancer further comprises allogenic stem cell transplantation.

The medical use herein described is formulated as a product as disclosed herein for use as a medicament for treatment of the stated diseases.

In this document and in its claims, the verb "to comprise" and its conjugations is used in its non-limiting sense to mean that items following the word are included, but items not specifically mentioned are not excluded. In addition, reference to an element by the indefinite article "a" or "an" does not exclude the possibility that more than one of the element is present, unless the context clearly requires that there be one and only one of the elements. The indefinite article "a" or "an" thus usually means "at least one".

The word "about" or "approximately" when used in association with a numerical value (e.g. about 10) preferably means that the value may be the given value (of 10) more or less 0.1% of the value.

### FIGURE LEGENDS

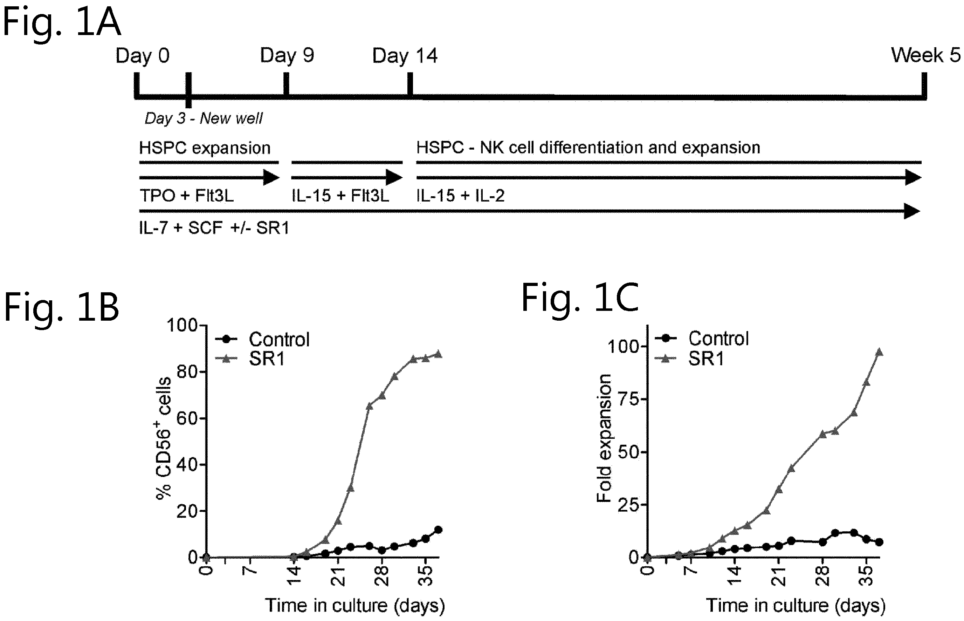

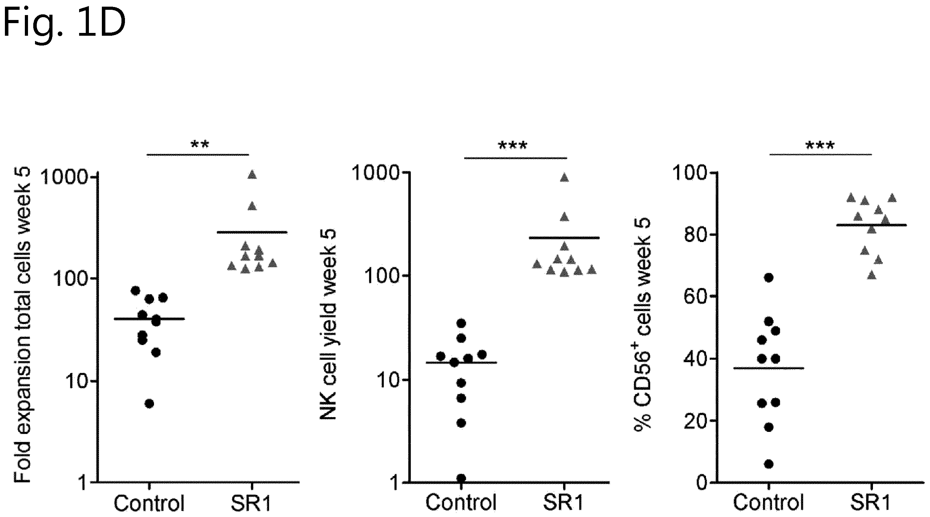

**Figure 1****. SR1 enhances expansion of peripheral blood-derived CD34+ HSPCs and improves NK cell differentiation.**
   (A) Representation of the *ex vivo* cytokine-based culture protocol used for the generation of NK cells.
   (B-D) Kinetics of differentiation (B) and expansion (C) of CD34+ HSPCs in our *ex vivo* culture protocol in the presence or absence of 2 µM SR1 in one representative donor, determined by FCM.
   (D) Summary of total cell expansion, NK cell yield (calculated by [total expansion x percentage of CD56+ cells]/100), and NK cell differentiation after 5 weeks of culture in our *ex vivo* culture protocol in the presence or absence of 2 µM SR1 in 12 donors, determined by FCM. ** *p<0.01, ***p<0.001,* Paired one-tailed student's t-test.

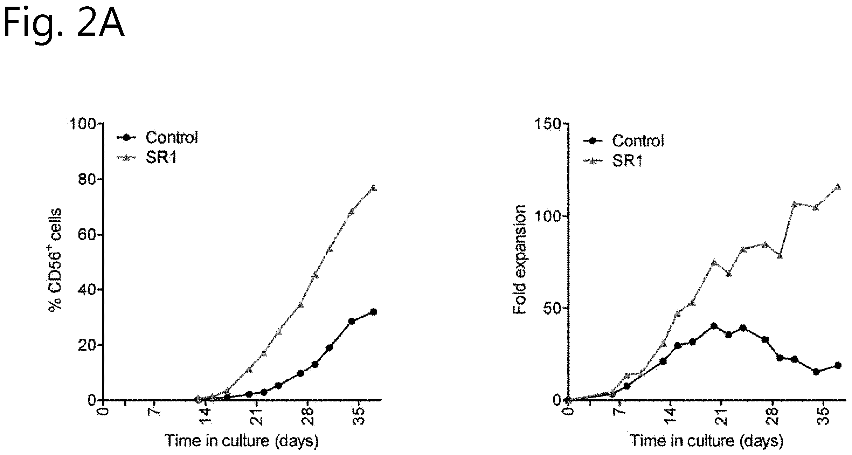

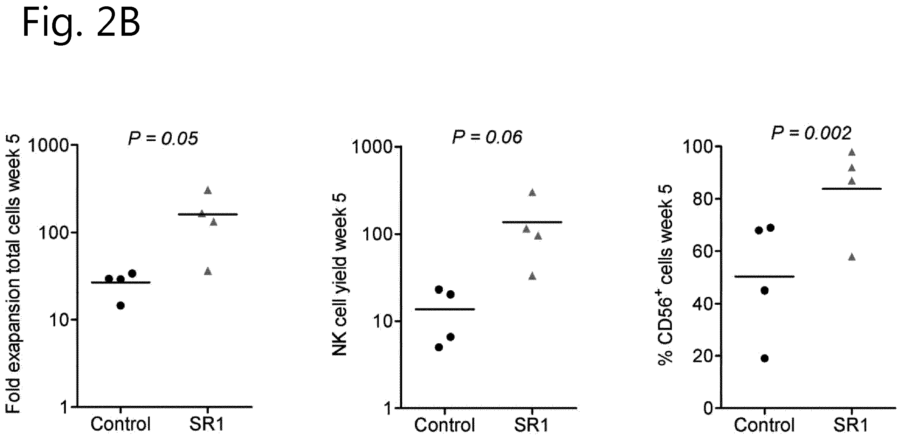

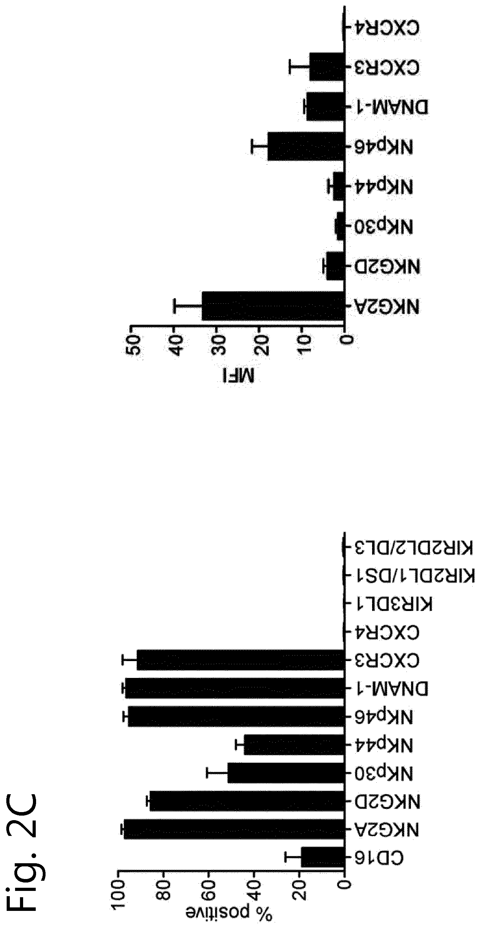

**Figure 2****. SR1 enhances expansion and NK cell differentiation of bone marrow-derived CD34+ HSPCs, thereby NK cells with a mature and active phenotype can be generated.**
   CD34+ HSPCs were isolated from bone marrow samples. The cells were cultured for 5 weeks in the *ex vivo* cytokine-based culture protocol described in the Materials and Method section and Figure 1A.
   (A) Kinetics of differentiation and expansion of CD56+ cells in the presence or absence of 2 µM SR1 from one representative donor.
   (B) Summary of total cell expansion, NK cell yield (calculated by [total expansion × percentage of CD56+ cells]/100), and NK cell differentiation after 5 weeks of culture in our *ex vivo* culture protocol in the presence or absence of 2 µM SR1 in 4 donors.
   (C) After 5 weeks of culture, expression of NK cell markers was analyzed using FCM. Expression levels and MFI of several NK cell specific surface antigens on viable CD56+ cells. Mean ± SEM of 2 - 3 different donors are shown. Paired one-tailed student's t-test.

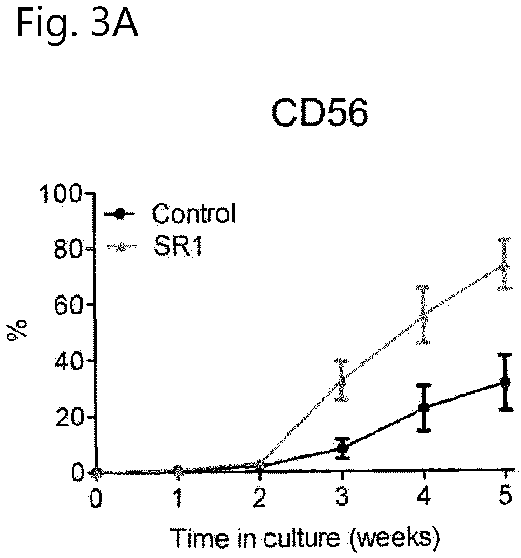

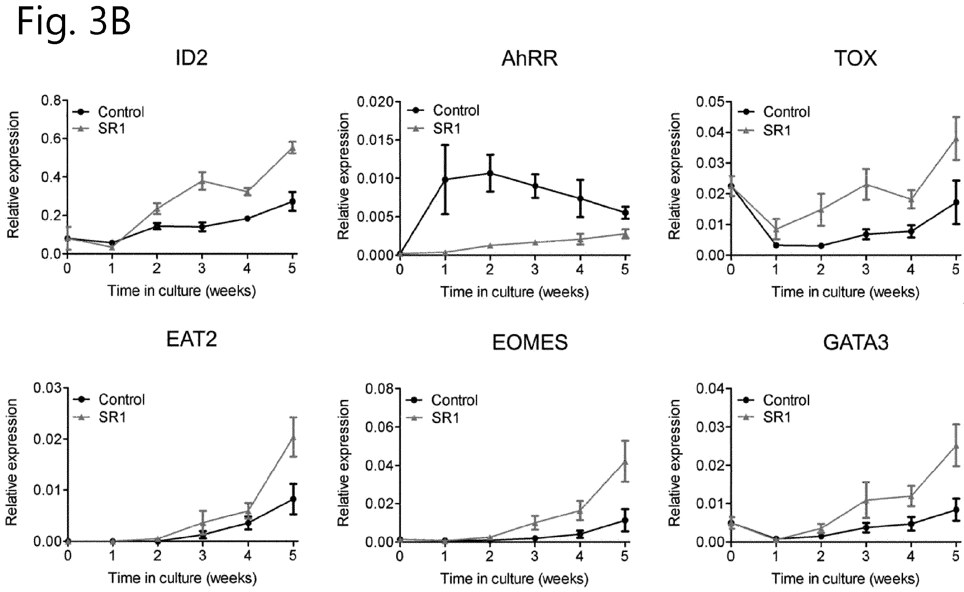

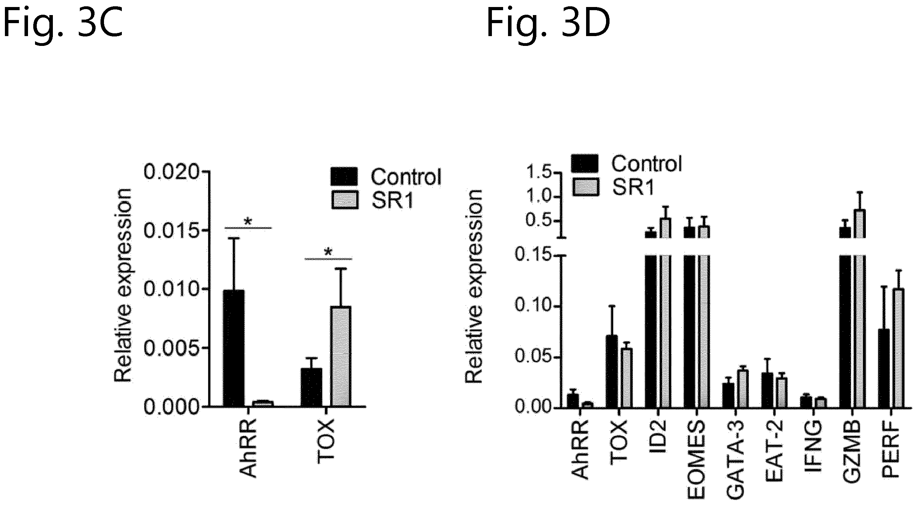

**Figure 3****. SR1 reduces expression of AhRR and increases expression of several transcription factors important for NK cell differentiation and NK cell effector functions.** Cells generated from CD34+ HSPCs in the presence or absence of SR1 were collected at different time points.
   (A) Kinetics of CD56 expression in the cultures used for weekly mRNA isolation were determined using FCM. Means ± SEM of 4 - 6 different donors.
   (B) Expression levels of AhRR and transcription factors relative to GAPDH at different time points during culture were determined using qRT-PCR. Means ± SEM of 4 - 6 different donors.
   (C) Expression levels of AhRR and TOX relative to GAPDH after 7 days of culture were determined using qRT-PCR. Means ± SEM of 6 different donors.
   (D) Expression levels of AhRR and transcription factors in CD56+ cells which were FACS sorted from cultures after 5 weeks of culture were determined using qRT-PCR. Means ± SEM of 4 different donors. *p<0.05. Paired one-tailed student's t-test.

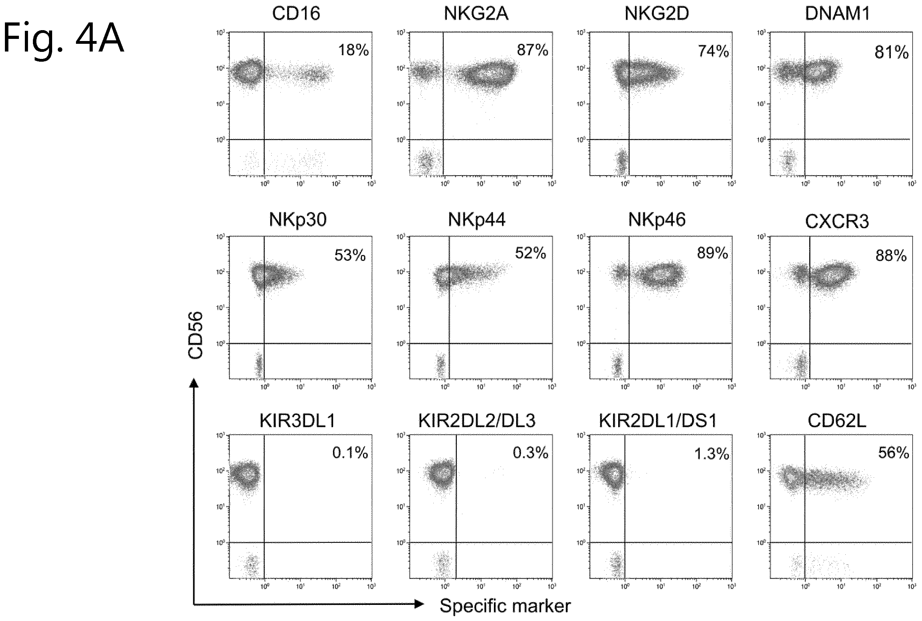

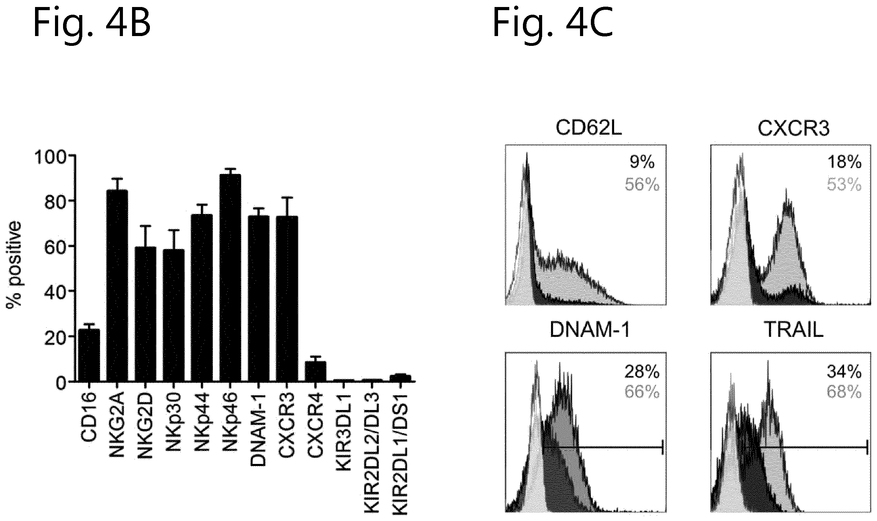

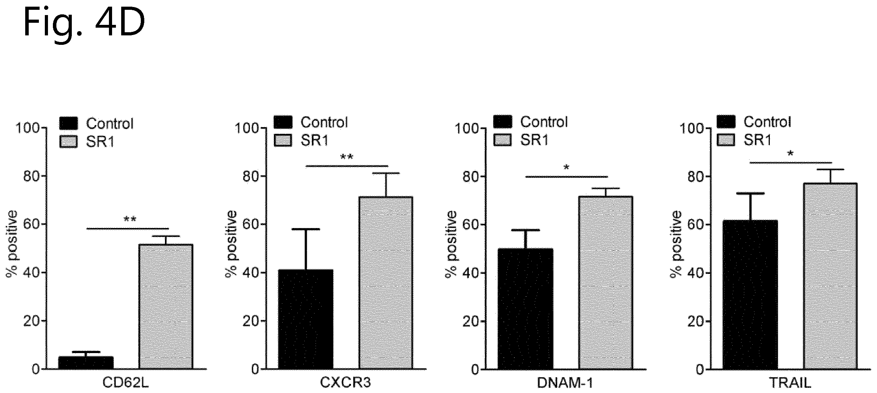

**Figure 4****: NK cells generated in the presence of SR1 have an active and mature phenotype.**
   NK cells were generated from CD34+ progenitor cells in the presence or absence of SR1. After 5 weeks a phenotypical analysis was performed by FCM.
   (A) Phenotype of a representative NK cell product. Density plot of live cells gated on forward scatter/side scatter.
   (B) Expression level of several NK cell specific surface antigens on live CD56+ NK cells from 3 - 8 different donors (Mean ± SEM) are shown.
   (C) Expression levels of NK cell-specific antigens on CD56+ NK cells generated in the presence (grey histograms) or absence (black histogram) of SR1 from one representative donor as compared to isotype controls (white histogram). Numbers represent % positive cells.
   (D) Expression levels of NK cell-specific antigens on CD56+ NK cells generated in the presence or absence of SR1 from 3 - 7 different donors (Mean ± SEM) are shown. *p<0.05, **p<0.01, Paired one-tailed student's t-test.

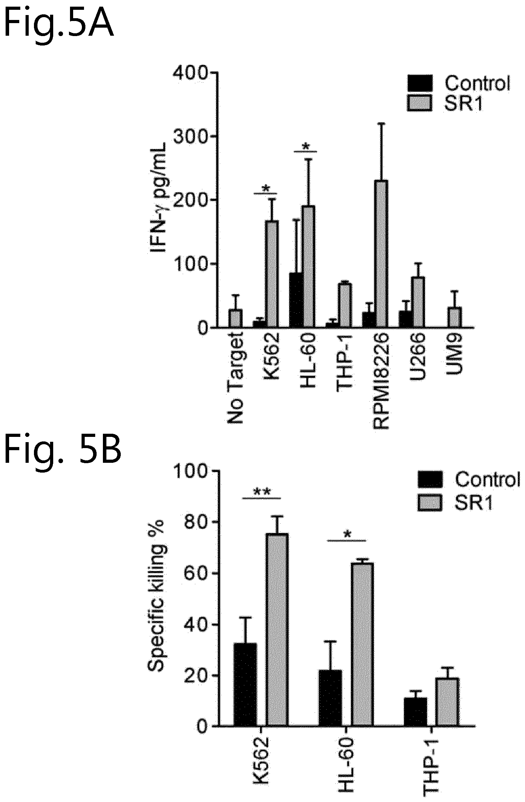

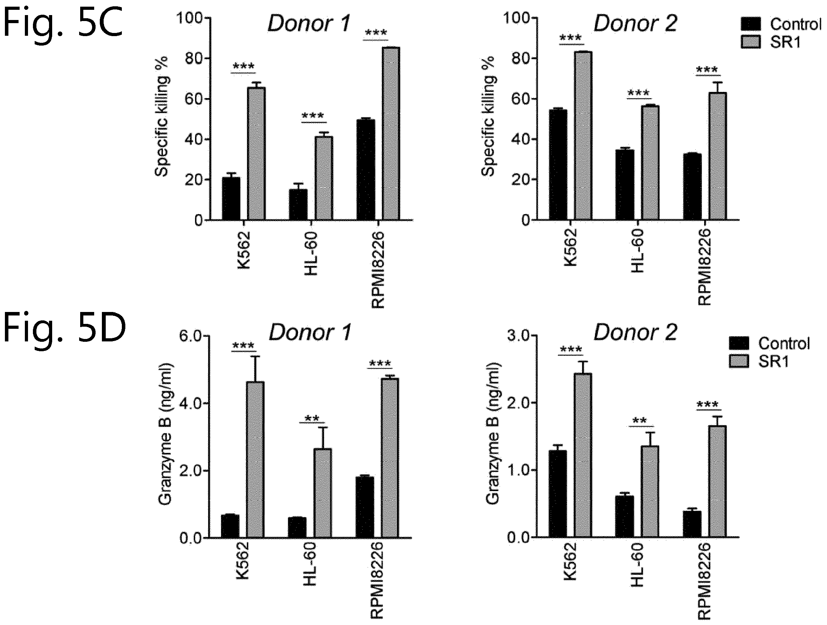

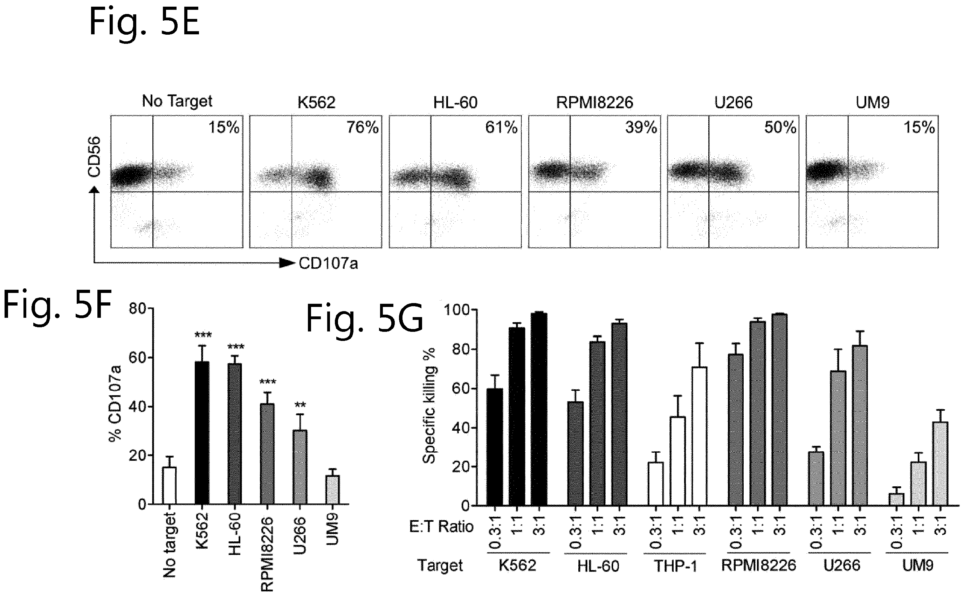

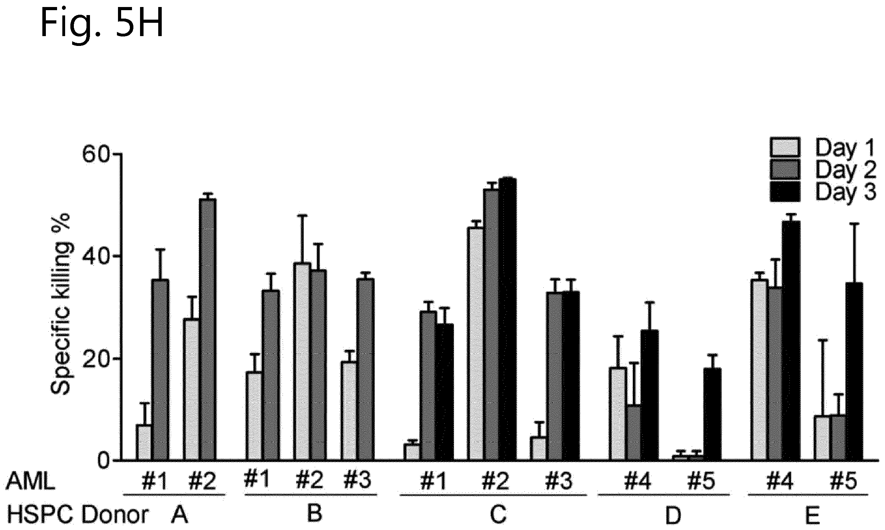

**Figure 5****. NK cells generated in the presence of SR1 are functionally active.**
   NK cells were generated from CD34+ HSPCs in the presence or absence of SR1. After 5 weeks the functional activity of the cells was investigated.
   (A-F) NK cells were co-cultured overnight with the leukemia cell lines K562, HL-60 or THP-1, or the MM cell lines RPMI8226, U266 or UM9 at an E:T ratio 0.3:1, 1:1 or 3:1.
   (A) IFN-γ levels were determined by ELISA in the co-culture supernatants of 1E+5 CD56+ NK cells and 1E+5 target cells. Means ± SEM of 3 - 4 donors generated in the presence or absences of SR1 are shown.
   (B) Specific killing of leukemia cell lines at an E:T ratio 0.3:1 was determined in a FCM-based cytotoxicity assay. Means ± SEM of 3 donors generated in the presence or absence of SR1 are shown.
   (C-D) After 5 weeks of culture, CD56+ NK cells were FACS sorted from the cultures, and co-cultured with leukaemia cell lines or the MM cell line RPMI8226 at an E:T ratio 1:1. Cultures were supplemented with IL-15 (5 ng/ml). (C) Specific killing of the cell lines was determined in a FCM-based cytotoxicity assay. Data are depicted as mean ± SD. (D) Granzyme B production by the CD56+ NK cells was measured by ELISA. Data are depicted as mean ± SD. (E-F) Degranulation of SR1-generated CD56+ cells after co-culture at an E:T ratio 1:1, determined by FCM as the percentage of CD107a expressing cells. One representative donor (E) and means ± SEM of 4 - 7 donors (F) are shown.
   (G) Specific killing of leukaemia or MM cell lines by SR1-generated NK cells at different E:T ratios was determined in a FCM-based cytotoxicity assay. Means ± SEM of 3 - 7 donors are shown.
   (H) Specific killing of primary AML cells by SR1-generated NK cells from 5 different patients (#1 AML-M2, #2 AML-M2, #3 AML-M4, #4 AML-M5, #5 AML-M0) was determined for 5 different HSPC donors. Specific killing was determined after 1, 2 and 3 days of co-culture in a FCM-based cytotoxicity assay at an E:T ratio of 3:1. Data are displayed as mean ± SD of triplicate samples. **p*<*0.05,* ***p*<*0.01,* ****p*<*0*.*001.* Paired one-tailed (A, B, F) or an unpaired two-tailed (C-D) student's t-test.

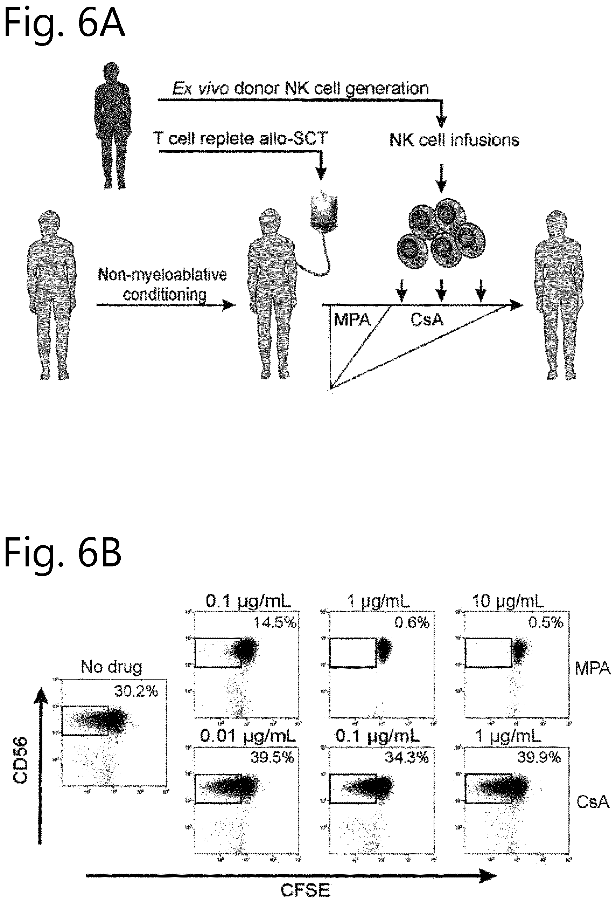

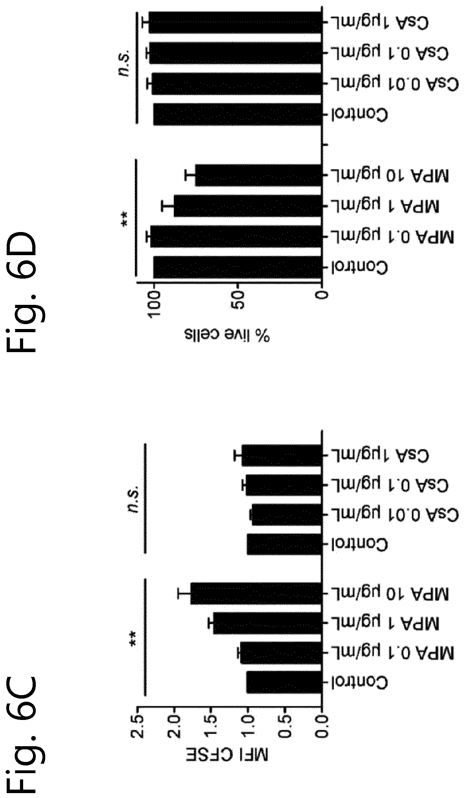

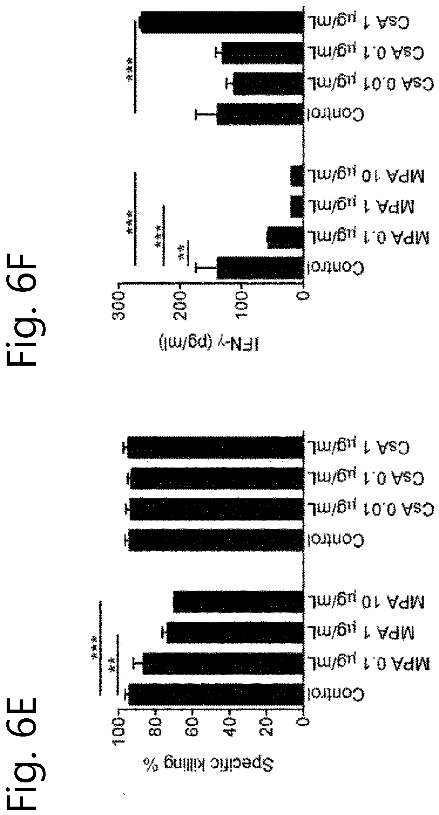

**Figure 6****. HSPC-NK cell viability, proliferation and function is inhibited by mycophenolic acid (MPA) but not by cyclosporin A (CsA).**
   (A) Suggested strategy for adoptive transfer of *ex vivo* generated SR1-NK cells after non-myeloablative allo-SCT. A patient is conditioned with non-myeloablative conditioning; subsequently, the patient receives a T cell replete stem cell graft. Ten percent of CD34+ cells of the donor graft are used for NK cell generation. To prevent GVHD, patients are treated with MPA and CsA. After cessation of MPA (around day 28 after transplantation), NK cells can be infused as a single infusion or multiple infusions.
   (B-F) The effect of immunosuppressive drugs used after non-myeloablative allo-SCT was investigated. NK cells were generated from CD34+ HSPCs in the presence of SR1. After 5 weeks, NK cells were cultured for 7 more days in the presence or absence of different dosages of MPA or CsA.
   (B) Proliferation of CFSE-labelled NK cells cultured in the presence of absence of drugs measured by CFSE dilution in a representative donor.
   (C) Proliferation of NK cells in the presence or absence of drugs measured as CFSE dilution. Mean MFI relative to MFI of control cells ± SEM of 4 different donors is shown.
   (D) The number of living cells determined by forward scatter/side scatter and exclusion of 7-AAD positive cells. Percentage of living NK cells cultured for 7 days in the presence or absence of drugs. Mean viability relative to control cells ± SEM of 4 different donors is shown.
   (E-F) 1E+5 CD56+ NK cells cultured in the presence or absence of MPA or CsA were co-cultured overnight with K562 cells in the presence of IL-15 (5 ng/ml), at an E:T ratio of 1:1. (E) Specific killing of K562 cells was determined in a FCM-based cytotoxicity assay. Data are depicted as mean ± SD and are representative of 2 different donors. (F) IFN-γ levels were measured in the supernatant after overnight co-culture using ELISA. Data are depicted as mean ± SD and are representative of 2 different donors. ** *p*<*0.01,* *** *p*<*0.001*, One-way ANOVA.

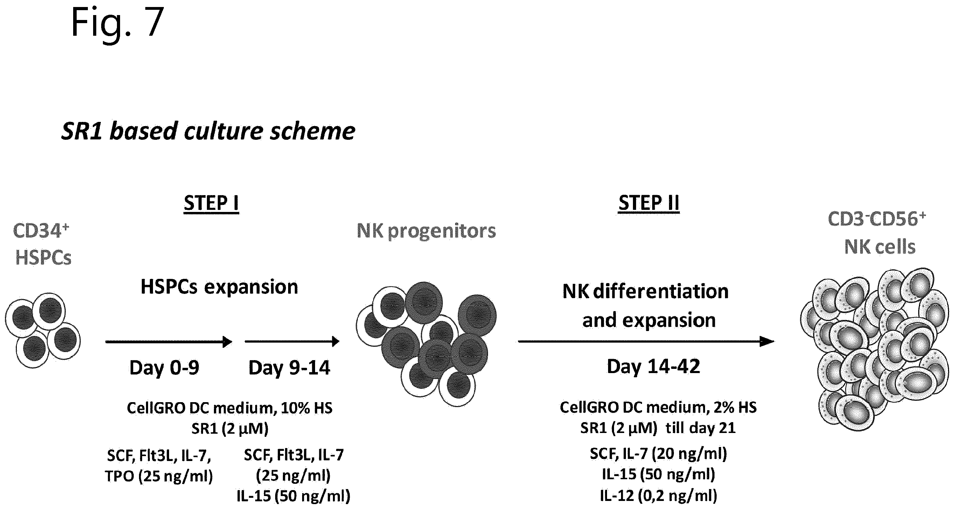

**Figure 7****. Innovative SR1-based culture protocol for improved *ex vivo* expansion of CD34+ HSPCs into functionally active NK cells.**
   Representation of the new *ex vivo* SR1-based culture protocol used for the generation of NK cells. In the expansion step, CD34+ cells are expanded and differentiated into NK progenitors using SCF, Flt3L, IL-7, TPO and an aryl hydrocarbon antagonist, preferably SR1. Thereafter, NK progenitors are further differentiated using IL-15, IL-12 and/or IL-2. The expansion and differentiation media as disclosed herein does not comprise a glycosaminoglycan/heparin, G-CSF, GM-CSF and/or IL-6.

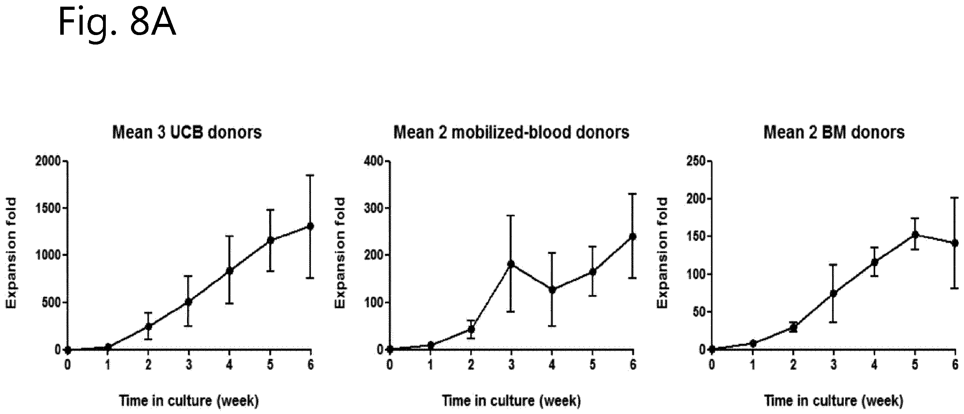

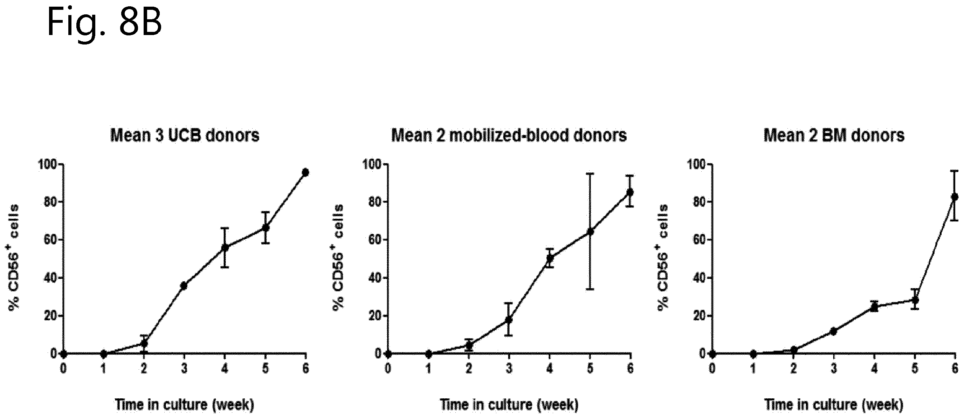

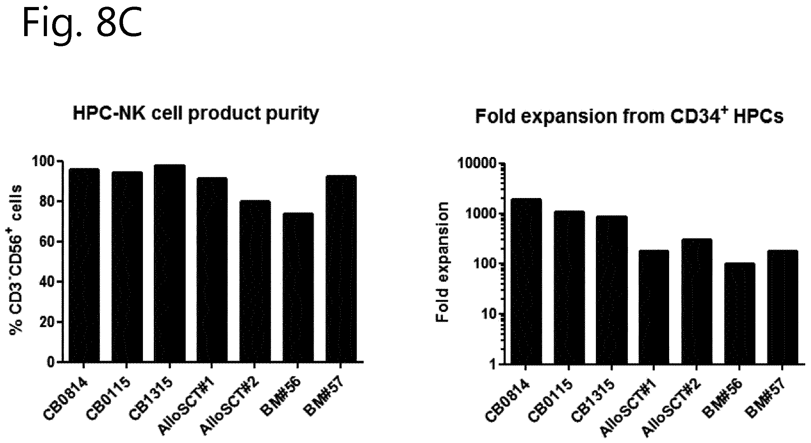

**Figure 8****. The inventive SR1-based culture protocol results in expansion and differentiation of UCB-, mobilized blood- and BM-derived CD34+ HSPCs into CD56+ NK cells.**
   (A-B) Kinetics of expansion (B) and differentiation (B) of CD34+ HSPCs enriched from UCB (n=3 donors), mobilized blood (n=2 donors) and BM (n=2 donors) in our inventive *ex vivo* culture protocol in the presence of 2 µM SR1 for 21 days.
   (C) Results of the final HPC-NK cell purity and fold expansion of the NK cell products generated from different CD34-positive HSPC sources in our inventive *ex vivo* culture protocol in the presence of 2 µM SR1.

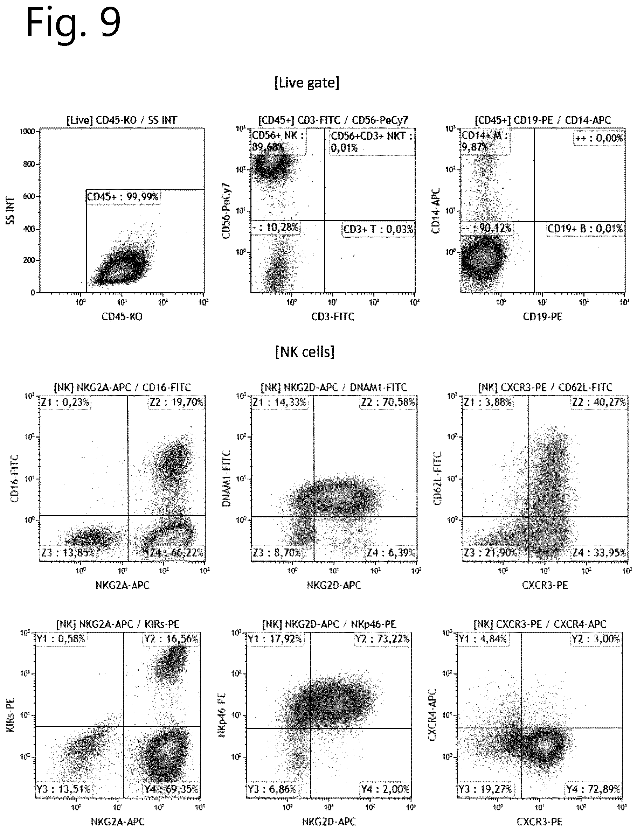

**Figure 9****. NK cells generated in the presence of SR1, IL-15 and IL-12 have an active and mature phenotype.**
   NK cells were generated from CD34+ progenitor cells in the presence SR1 using our inventive *ex vivo* culture protocol depicted in figure 7. After 6 weeks a phenotypical analysis was performed by FCM. In the upper panel CD56+ purity is shown in comparison with absence of CD3+ T cells, CD19+ B cells and low percentage of CD14+ monocytic cells. In the two lower panels the phenotype of viable CD56+ cells is shown for a panel of NK cell associated differentiation, activation and functional markers.

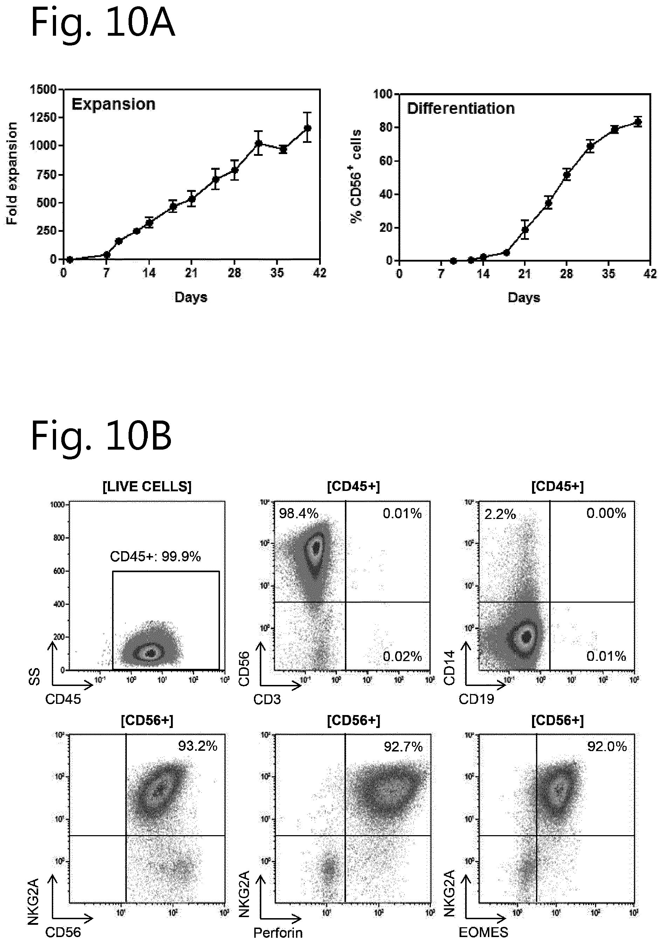

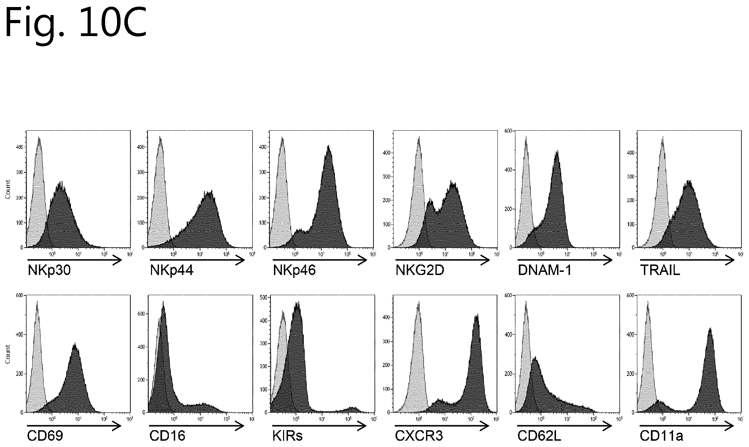

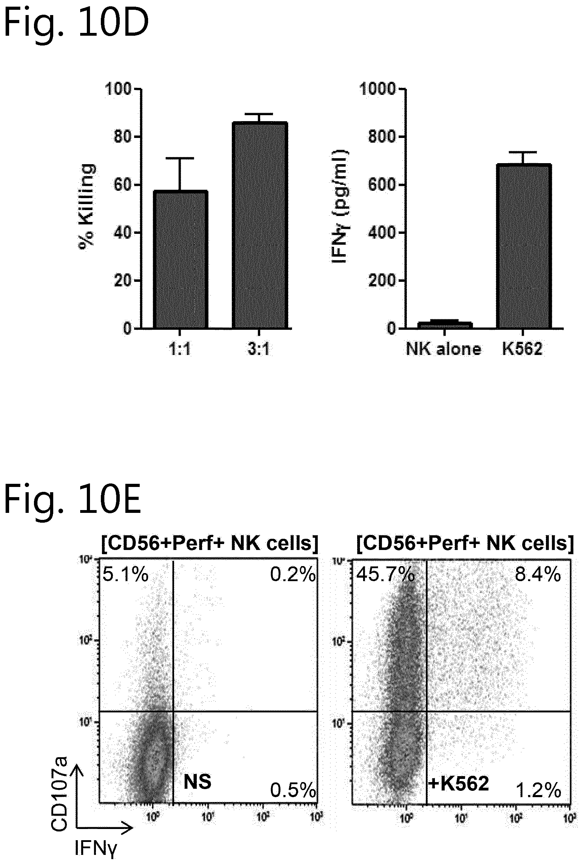

**Figure 10****. NK cells generated in the presence of SR1, IL-15 and IL-12 have an activated phenotype and display potent cytolytic and IFN-γ producing functions.**
   NK cells were generated from CD34+ UCB progenitor cells in the presence SR1 using our inventive *ex vivo* culture protocol depicted in figure 7. After 6 weeks a phenotypical analysis was performed by FCM. (A) Fold expansion and percentage of CD45+CD56+CD3- cells during culture of SR1-expanded UCB-derived HSPC-NK cell products. (B) Representative flow cytometric analysis of UCB-derived HSPC-NK cell products. FACS plots illustrate that final products mostly comprises of CD56⁺ cells (>85%) and CD14⁺ cells (0-15%) and < 0.5% CD19+ B cells. Contaminating T cells were virtually absent (< 0.05%). Among CD56⁺ cells, conventional NK cells can be identified by Perforin, NKG2A and EOMES expression. Remaining CD56+ ILC3 cells can be identified by RORyt expression.
   (C) Representative flow cytometric analysis of SR1-expanded HSPC-NK cell products in terms of activation, maturation, homing and adhesion markers. FACS plots are gated on total CD56⁺ cells.
   (D) Cytolytic activity and IFNγ production capacity of SR1-expanded HSPC-NK cells against K562 cells at low E:T ratios (mean ± SEM of 3 products). (E) Representative analysis of HSPC-NK cell reactivity against K562 cells at the single cell level by flow cytometry. NS = non stimulated.

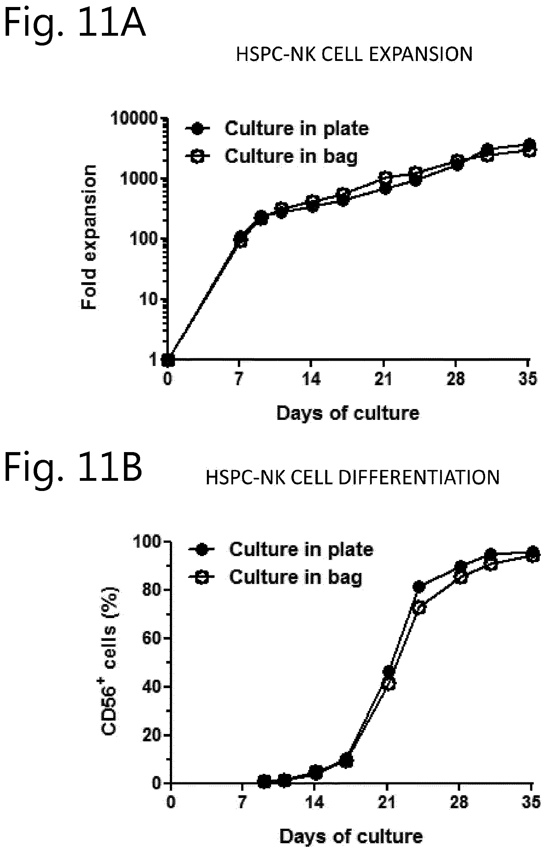

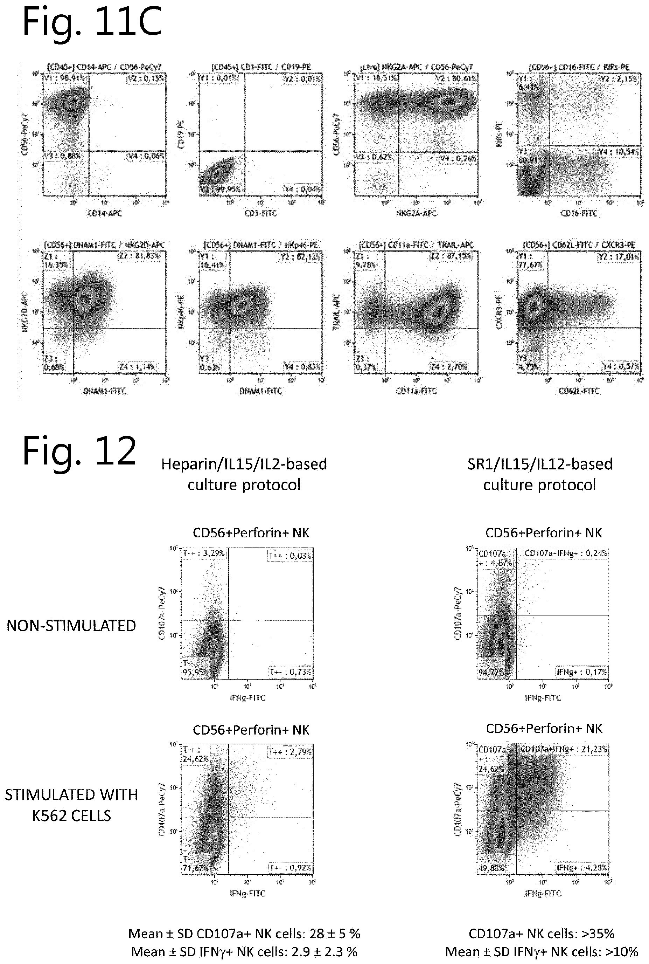

**Figure 11****. NK cells generated in the presence of SR1, IL-15 and IL-12 can be efficiently manufactured in closed-system cell culture bag.**
   NK cells were generated from CD34+ UCB progenitor cells in the presence SR1 using our inventive *ex vivo* culture protocol depicted in figure 7. CD34+ UCB cells were either cultured in 6-well plates or closed-system VueLife^{™} culture bag in parallel (A) The total cell expansion after 5 weeks of culture was similar between plates and the bag, which was >2,500 fold. (B) The percentage CD56+ cells at 5 weeks of culture was similar between plates and bag, which was 95%. (C) Flow cytometric analysis of the bag-cultured HSPC-NK cell product demonstrated that the SR1-expanded final product comprises >80% CD56⁺NKG2A⁺LFA-1⁺ NK cells with high expression of NKG2D, DNAM-1, CXCR3 and TRAIL.
**Figure 12****. NK cells generated in the presence of SR1, IL-15 and IL-12 display high degranulation and IFN-γ producing functions.** Degranulation and IFNγ secretion of SR1-expanded CD56+Perforin+ NK cells after co-culture at an E:T ratio 1: 1 with K562 target cells was determined at the single cell level by flow cytometry. The frequency of CD107a⁺ degranulating and IPNγ-positive NK cells obtained by our inventive *ex vivo* culture protocol is >35% and >10%, respectively, which is significantly better than obtained by an Heparin/IL15/IL2-based protocol.

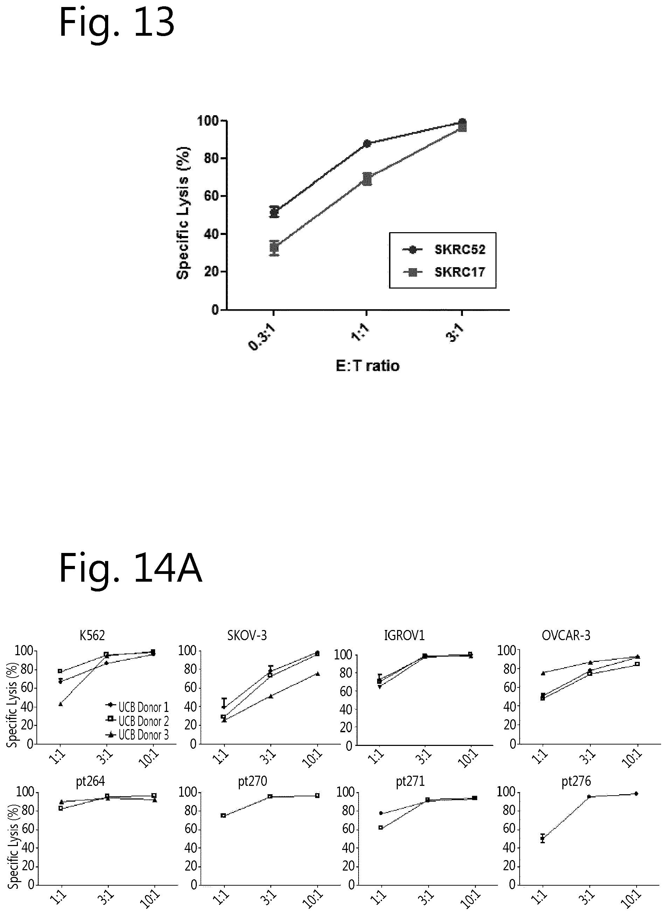

**Figure 13****. SR1-expanded HSPC-NK cells efficiently kill renal cell carcinoma cells in vitro.** Percentage specific lysis of two different patient-derived RCC mono-layers at low E:T ratios.

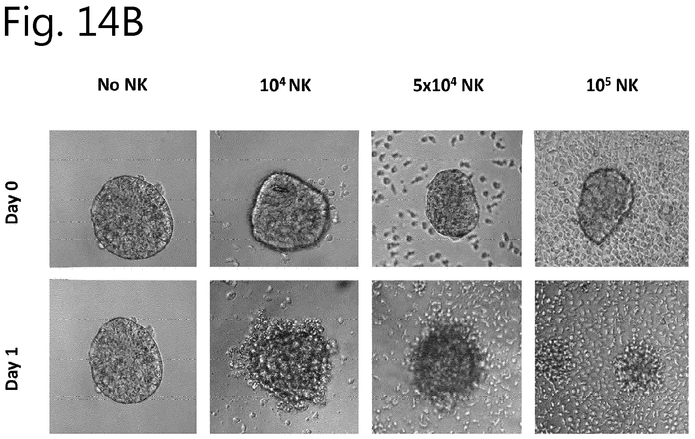

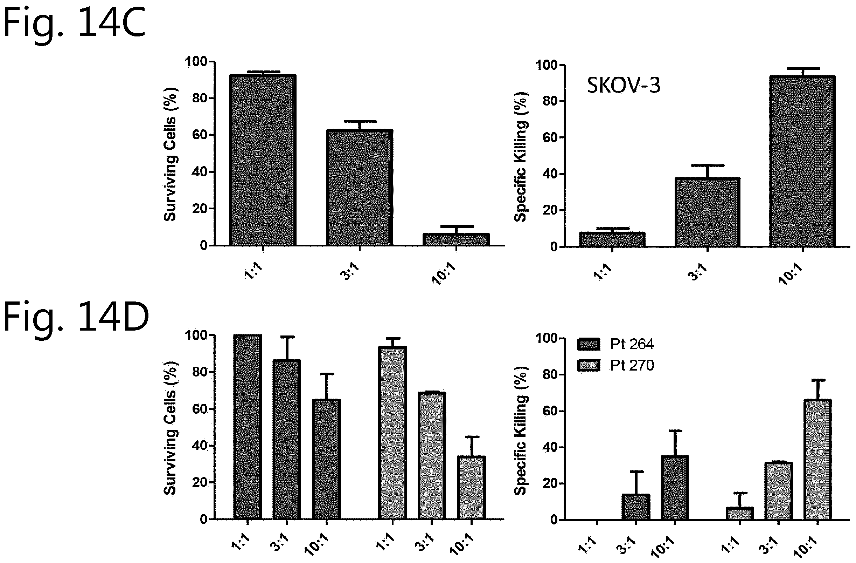

**Figure 14****. SR1-expanded HSPC-NK cells efficiently kill ovarian cancer cell lines and spheroids in vitro.** (A) Percentage specific lysis of different ovarian cancer (OC) cell lines (upper row) and patient-derived OC mono-layers cultured from fresh patient ascites (lower row) in comparison with K562 cells (i.e. positive control). All experiments are performed with 1-3 different NK cell donors. (B-D) HSPC-NK mediated killing of *in* vivo-like tumor spheroids of the SKOV-3 cell line (B, microscopic pictures and C, killing at increasing E:T ratios) and peritoneal tumor cells from two high-grade serous OC patients (D killing at increasing E:T ratios).

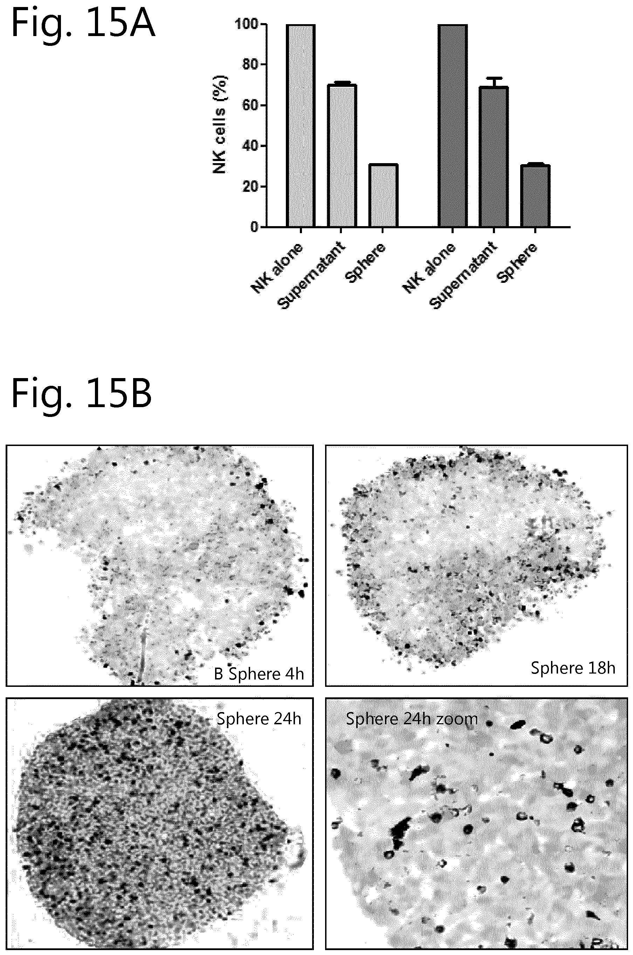

**Figure 15****. SR1-expanded HSPC-NK cells efficiently infiltrate tumor spheroids in vitro.** (A) SKOV-3 ovarian cancer (OC) spheroids were co-cultured with HSPC-NK cells for 24 hours at different E:T ratios. Thereafter, OC spheroids were collected, and non-infiltrated NK cells (i.e. supernatant fraction) were removed by washing. Solid spheres were disintegrated (i.e. sphere fraction) and amount of viable CD56+NKG2A+ NK cells were quantified by flow cytometry. Notably, the percentage of infiltrating NK cells was 30% within 24 hours at both E:T ratios. (B) Confocal microscopy imaging showed that after 24 hours an increasing amount of CD56+ HSPC-NK cells deeply infiltrated the SKOV-3 GFPluc spheroid.

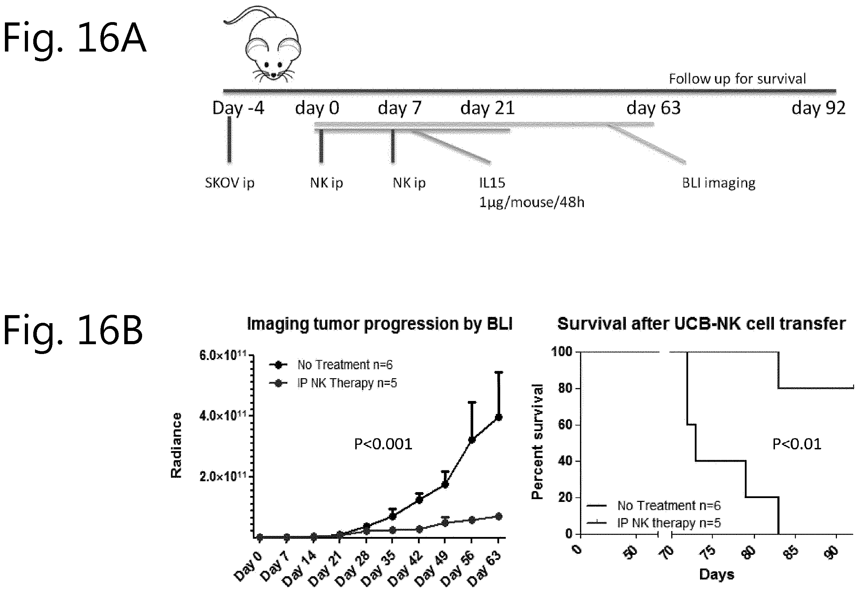

**Figure 16****. SR1-expanded HSPC-NK cells efficiently target SKOV-3 OC tumors in vivo.** (A) NSG mice were injected IP with SKOV-3 cells, followed by 2 HSPC-NK cell infusions, supportive rhIL-15 injections, and weekly bioluminescence measurements. SKOV-3 tumors develop in omentum, ovaries and peritoneum. (B) Luminescence of the NSG mice intraperitoneally injected with SKOV-3 GFPluc cells. A rapid increase in tumor growth in the no treatment group is shown. In contrast, the tumor growth was effectively decreased in SKOV-3 bearing NSG mice that received HSPC-NK cell injections intraperitoneally. (C) Survival of the same mice is shown. Notably, the survival of HSPC-NK cell treated mice was significantly prolonged up to day 92 after tumor injection (i.e. end of the experiment). Statistical analysis was performed using Two-way ANOVA with Bonferoni post-hoc test or Mantel_cox survival analysis.

### EXAMPLES

The present invention is further described by the following examples which should not be construed as limiting the scope of the invention.

Unless stated otherwise, the practice of the invention will employ standard conventional methods of molecular biology, virology, microbiology or biochemistry. Such techniques are described in Sambrook et al. (1989) Molecular Cloning, A Laboratory Manual (2nd edition), Cold Spring Harbor Laboratory, Cold Spring Harbor Laboratory Press; in Sambrook and Russell (2001) Molecular Cloning: A Laboratory Manual, Third Edition, Cold Spring Harbor Laboratory Press, NY; in Volumes 1 and 2 of Ausubel et al. (1994) Current Protocols in Molecular Biology, Current Protocols, USA; and in Volumes I and II of Brown (1998) Molecular Biology LabFax, Second Edition, Academic Press (UK); Oligonucleotide Synthesis (N. Gait editor); Nucleic Acid Hybridization (Hames and Higgins, eds.).

### Example 1. The aryl hydrocarbon receptor antagonist StemRegenin 1 (SR1) improves in vitro generation of highly functional NK cells from CD34+ hematopoietic stem and progenitor cells.

### Abstract

Early natural killer (NK) cell repopulation after allogeneic stem cell transplantation (allo-SCT) has been associated with reduced relapse rates without an increased risk of graft-versus-host disease (GVHD), indicating that donor NK cells have specific anti-leukemic activity. Therefore, adoptive transfer of donor NK cells is an attractive strategy to reduce relapse rates after allo-SCT. Since NK cells of donor origin will not be rejected, multiple NK cell infusions could be administered in this setting. However, isolation of high numbers of functional NK cells from transplant donors is challenging. Hence, we developed a cytokine-based *ex vivo* culture protocol to generate high numbers of functional NK cells from G-CSF mobilized CD34+ stem and progenitor cells (HSPCs). In this study, we demonstrate that addition of aryl hydrocarbon receptor (AhR) antagonist SR1 to our culture protocol potently enhances expansion of CD34+ HSPCs, and induces expression of NK cell associated transcription factors promoting NK cell differentiation. As a result, high numbers of NK cells with an active phenotype can be generated using this culture protocol. These SR1-generated NK cells exert efficient cytolytic activity and IFN-γ production towards acute myeloid leukemia (AML) and multiple myeloma (MM) cells. Importantly, we observed that NK cell proliferation and function is not inhibited by cyclosporin A (CsA), an immunosuppressive drug often used after allo-SCT. These findings demonstrate that SR1 can be exploited to generate high numbers of functional NK cells from G-CSF mobilized CD34+ HSPCs, providing great promise for effective NK cell based immunotherapy after allo-SCT.

### Introduction

Natural killer cells (NK cells) are CD3-CD56+ lymphocytes, which are part of the innate immune system and play an important role in the defense against virus-infected and transformed cells. NK cell activation and subsequent killing of target cells is regulated by a balance in their expression levels of inhibitory receptors, including the killer-immunoglobulin like receptors (KIRs) and CD94/NKG2A heterodimer, versus activating receptors, such as DNAX accessory molecule-1 (DNAM-1), natural cytotoxicity receptors (NCRs) and NKG2D. In homeostasis, NK cells are inhibited by their inhibitory receptors recognizing self human leukocyte antigen (HLA) class I molecules and/or HLA-E molecules presenting conserved HLA class I leader sequences. However, an NK cell-mediated anti-tumor effect can be induced by up-regulation of activating ligands or down-regulation of HLA class I molecules on tumor cells. In addition, in the setting of haploidentical allogeneic stem cell transplantation (allo-SCT), donor NK cells may lack expression of inhibitory KIRs for recipient HLA class I molecules and hence be activated. This phenomenon is called missing-self recognition and can contribute to the curative graft-versus-tumor (GVT) effect [1].

Because of their ability to kill tumor cells, NK cells are considered potent effectors for adoptive immunotherapy against cancer. So far, promising results have been obtained by infusion of haploidentical NK cells after immunosuppressive chemotherapy in adult and childhood acute myeloid leukemia (AML) [2-4]. However, a limitation in these studies is the relatively low NK cell numbers that can be enriched from aphaeresis products for multiple infusions. Furthermore, contaminating alloreactive T cells risk the induction of graft-versus-host disease (GVHD), especially when IL-2 or IL-15 are co-administrated to boost NK cell survival and expansion. In order to generate high numbers of allogeneic NK cells completely devoid of T cell contamination, a Good Manufacturing Practice (GMP)-compliant, cytokine-based *ex vivo* culture protocol has been developed by our group [5,6]. Using this procedure, CD34+ hematopoietic stem and progenitor cells (HSPCs) isolated from umbilical cord blood (UCB) can be expanded over 2000-fold in large-scale bioreactors into a mixture of immature and mature NK cells with a purity >80%. Pre-clinical studies conducted in NOD/SCID-IL2Rynull mice demonstrated that these HSPC-NK cells have BM homing capacity, display IL-15-driven in vivo expansion, and prolong survival of leukemia-bearing mice [7]. Currently, administration of this HSPC-NK cell product following immunosuppressive chemotherapy is being investigated in a phase I clinical trial in older AML patients who are not eligible for allo-SCT (see www.trialregister.nl and search for 2818).

In HLA-matched non-myeloablative and T cell-depleted allo-SCT, early NK cell repopulation has been associated with decreased relapse rates, without increasing GVHD incidence [8,9]. Moreover, high NK cell numbers in stem cell grafts have been associated with a decreased incidence of GVHD [10]. In addition, transplants from donors with KIR-B haplotypes, containing several activating KIRs, led to lower rates of relapse and improved survival [11-13]. For these reasons, it would be highly valuable to exploit HSPC-NK cell products for adoptive immunotherapy after allo-SCT. Since NK cells of donor origin will not be rejected, multiple NK cell infusions without the need for immunosuppressive chemotherapy to prevent rejection, could be administered after allo-SCT. Consequently, these cells may potentially induce long-term GVT effects. However, to obtain large numbers of NK cells from donor origin, peripheral blood (PB) or bone marrow (BM)-derived CD34+ HSPCs, which have a lower expansion potential compared to UCB-derived CD34+ HSPCs, should be expanded and differentiated into NK cells. Recently, it was described that expansion of CD34+ HSPCs can be enhanced by inhibition of the aryl hydrocarbon receptor (AhR) using the antagonist StemReginin1 (SR1) [14]. AhR is a ligand-inducible transcription factor, which plays an important role in biological responses towards xenobiotic agents such as digoxin [15,16]. Yet, it has become clear that AhR also has multiple naturally occurring ligands, like tryptophan metabolites and dietary compounds [15]. Furthermore, AhR was hypothesized to regulate differentiation of multiple immune cells including dendritic cells [17,18], regulatory T cells [19,20], γδ T cells [21] and Th17 cells[22]. Regarding NK cell differentiation, it has been observed that antagonism of AhR or silencing of AhR gene expression promotes differentiation of tonsillar IL-22-producing IL-1R1^{hi} human ILC3s to CD56^{bright}CD94+ interferon (IFN)-g-producing cytolytic mature NK cells expressing EOMES and TBET/TBX21 [23]. Based on these findings, we hypothesized that addition of SR1 to our culture system might improve expansion of CD34+ HSPCs, and differentiation of these cells into NK cells. We found that SR1 not only enhances expansion of CD34+ HSPCs, but also up-regulates the expression of early and late NK cell specific transcription factors such as TOX, ID2, EOMES, GATA-3 and EAT-2, thereby potentiating differentiation of SR1-expanded CD34+ cells into NK cells. These SR1-induced NK cells have a high purity, express high levels of activating receptors and efficiently target AML and multiple myeloma (MM) cells. Importantly, proliferation and cytolytic functions of SR1-induced HSPC-NK cells are not inhibited by cyclosporin A (CsA), in contrast to mycophenolic acid (MPA), which is used only shortly after allo-SCT, facilitating multiple infusions relatively shortly after allo-SCT. Therefore, our SR1 culture system holds great promise for future donor HSPC-NK cell adoptive immunotherapy after allo-SCT to boost anti-tumor and anti-viral immunity, leading to prolonged relapse-free survival.

### Materials and Methods

### Cell lines

Cell lines (K562, THP1, HL-60, U266, UM9, RPMI8226) were cultured in Iscove's modified Dulbecco's medium (IMDM; Invitrogen, Carlsbad, CA, USA) containing 50 U/mL penicillin, 50 µg/mL streptomycin and 10% fetal calf serum (FCS; Integro, Zaandam, The Netherlands).

### Isolation of CD34+ stem and progenitor cells

Bone marrow samples were collected from healthy donors after written informed consent. Bone marrow-derived mononuclear cells (BM-MNCs) were isolated by Ficoll-hypaque (1.077 g/mL; GE Healthcare, Uppsala, Sweden) density gradient centrifugation. Peripheral blood-derived mononuclear cells (PB-MNCs) were obtained from aphaeresis material from stem cell donors who were treated with G-CSF (Neupogen^{®}) 10 µg/kg/day subcutaneously for 5 to 6 days, after written informed consent. CD34+ HSPCs were isolated using anti-CD34 immunomagnetic beads (Miltenyi Biotech, Bergisch Gladbach, Germany) according to manufacturer's instructions. CD34+ HSPCs were directly used for NK cell generation.

### Ex vivo expansion of CD34+HSPCs, and differentiation into NK cells

CD34+ HSPCs were plated into 24-well or 6-well tissue culture plates (Corning Incorporated, Corning, NY, USA). Cells were expanded during 9 or 10 days with a high-dose cytokine cocktail (Expansion cocktail I) consisting of 25 ng/mL IL-7 (Immunotools), 25 ng/mL SCF (Immunotools), 25 ng/mL TPO (Cellgenix) and 25 ng/mL Flt3L (Immunotools). From day 9 or 10 until day 14 or 15, TPO was replaced by 20 ng/mL IL-15 (Miltenyi). After day 14 or 15, cell differentiation was initiated by replacing Expansion cocktail I by a new high-dose cytokine cocktail (Differentiation cocktail) consisting of 20 ng/mL IL-7, 20 ng/mL SCF, 20 ng/mL IL-15 and 1000 U/mL IL-2 (Proleukin^{®}; Chiron, München, Germany). Where mentioned, 2 µM StemRegenin1 (SR1; Cellagen Technology, San Diego, CA, USA) was added to the culture medium (see Figure 1A). Cells were cultured in Cellgro GMP DC medium (Cellgenix, Freiburg, Germany) supplemented with 10% human serum (HS; Sanquin Bloodbank, Nijmegen, The Netherlands) and a low dose cytokine cocktail consisting of 250 pg/mL G-CSF (Filgrastim, "Neupogen" - Amgen Corp. USA), 10 pg/mL GM-CSF and 50 pg/mL IL-6 (both Immunotools, Friesoythe, Germany). During the first 14 or 15 days of culture, low molecular weight heparin (Clivarin^{®}; Abbott, Wiesbaden, Germany) was added to the medium in a final concentration of 20 µg/mL. Freshly isolated CD34+ cells were plated at a concentration of 1 - 4E+5/mL. After 3 days of culture, cells were transferred to a new plate in order to deplete for stromal cells. From day 14 or 15 onward, cell counts were kept above 2E+6 cells/mL. Cell cultures were refreshed with at least 30% new medium every 2 to 3 days. Cultures were maintained at 37°C, in 95% humidity, and 5% CO2. NK cells were used in experiments after 5 weeks of culture.

### RNA isolation and real-time quantitative RT-PCR (qRT-PCR)

Total RNA from 0.5 - 2E+5 cells, collected weekly from HSPC-NK cell cultures, was isolated using the Quick-RNATM MicroPrep Kit (Zymo Research, CA, USA). Next, cDNA was synthesized using M-MLV-reverse transcriptase (Invitrogen) in a standard reaction as described before [24], after which real-time PCR was performed using the following Taqman Gene expression assays (Applied Biosystems, Forster City, CA, USA): AhRR (Hs01005075_m1); TOX (Hs01055573_m1); ID2 (Hs04187239_m1); EOMES (Hs00172872_m1); GATA3 (Hs00231122_m1); SH2D1B (Hs01592483_m1); IFNG (Hs00989291_m1); GZMB (Hs01554355_m1); PRF1 (Hs99999108_m1). For all genes, Ct values were normalized to GAPDH (Hs02758991_g1) by calculating ΔCt = Ct_{target gene} - Ct_{GAPDH} per sample. Finally, gene expression levels were quantified relative to GAPDH as follows: 2^(-[ΔCt]).

### Flow cytometry (FCM)

Cell numbers and expression of cell-surface markers were determined by FCM. Antihuman CD45-ECD (J.33, Beckman Coulter, Woerden, The Netherlands) and anti-CD56-PC7 (HCD56, Biolegend, San Diego, CA, USA) antibodies were used to follow cell number and NK cell differentiation during culture using the Coulter FC500 flow cytometer (Beckman Coulter). The population of viable CD45+ cells was determined by exclusion of 7-AAD (Sigma, St Louis, MO, USA) positive cells. For phenotypical analysis, cells were incubated with antibodies in FCM buffer (PBS/0.5% bovine serum albumin (BSA; Sigma) for 30 minutes at 4°C. After washing, cells were resuspended in FCM buffer and analyzed. The following conjugated monoclonal antibodies were used for NK cell phenotyping: anti-NKG2A-PE (Z199; Beckman Coulter), anti-DNAM-1-FITC (DX11; BD Biosciences Pharmingen, Breda, The Netherlands), anti-CD16-FITC (3G8), anti-CD3-FITC (UCHT1), anti-NKG2D-PE (1D11), anti-NKp30-PE (P30-15), anti-NKp44-PE (P44-8), anti-NKp46-PD (9E5), anti-CD158b-PE (Dx27), anti-CD158e-PE (Dx9), anti-CD158a/h-PE (HP-MA4), anti-CD62L-PE (DREG56), anti-CD253-PE (RIK-2), anti-CXCR3-PE (G025H7), anti-CXCR4-PE (12G5), anti-IgG1-PE (MOPC-21), anti-IgG2a-PE (MOPC-173), anti-IgG2b-PE (MCP-11), anti-IgG1-FITC (MOPC-21; all from Biolegend).

### Fluorescence-activated cell sorting (FACS) of HSCP-NK cells

After 5 weeks of culture, CD56+ cells were isolated from the total cultured cells. For this purpose cells were stained for 15 minutes at 4°C using the appropriate concentration of anti-CD56-PEcy7 (CD56-PC7 (HCD56, Biolegend). Cells were washed and resuspended in FACS buffer at a concentration of 1.5E+6/mL, and subsequently sorted at the FACSAria Cell Sorter (BD Biosciences).

### FCM-based cytotoxicity assays

Cell lines were labeled with 1 µM carboxyfluorescein diacetate succinimidyl ester (CFSE; Molecular Probes Invitrogen, Eugene, OR, USA) and primary AML blasts were labeled with 1.5 µM CFSE, both in a concentration of 1E+7/mL for 10 minutes at 37°C. The reaction was terminated by adding an equal volume of FCS. After washing, cells were resuspended in IMDM/10% FCS to a final concentration of 3E+5/mL. Target cells (3E+4) were co-cultured in triplicate with effector cells at different effector:target (E:T) ratios in a total volume of 200 µL IMDM/10% FCS in 96-wells round-bottom plates (Corning Incorporated). Effector cells and target cells alone were plated out in triplicate as controls. In experiments with primary AML blasts, AML blasts were derived from bone marrow samples from 5 patients at the time of diagnosis and were supplemented with IL-3 (50 ng/mL; Cellgenix), SCF (25 ng/mL), Flt3L (20 ng/mL), GM-CSF (100 ng/mL), G-CSF (100 ng/mL) and IL-15 (5 ng/mL). To measure degranulation of NK cells, anti-CD107a-PC7 (H4A3, Biolegend) was added to the co-culture. After overnight co-culture at 37°C, 50 µL supernatant was discarded and 50 µL Coulter^{®} Isoton^{®} II Dilutent containing 0.2 µL 7-AAD was added instead. Cells were harvested and the number of viable target cells was quantified by FCM by gating on forward scatter and side scatter and exclusion of 7-AAD positive cells. Target cell survival was calculated as follows: % survival = ([absolute number of viable CFSE+ target cells co-cultured with NK cells]/[absolute number of viable CFSE+ target cells cultured in medium]) × 100%. The percentage specific lysis was calculated as follows: % lysis = (100-[% survival]), as described earlier by Jedema et al. [25,26] Degranulation of NK cells during overnight co-culture was determined as the percentage of CD107a expressing cells measured by FCM.

### Enzyme-linked immunosorbent assays

The production capacity of IFN-γ and Granzyme B by NK cells were evaluated by ELISA according to manufacturer instructions (IFN-γ: Pierce Endogen, Rockfore, IL, USA; Granzyme B; Mabtech, Sweden). To this end, NK cells (1E+5) were co-cultured in triplicate with target cells (1E+5) in a total volume of 200 µL IMDM/10% FCS in 96-wells round-bottom plates (Corning Incorporated). NK cells alone were plated out in triplicate as controls. After incubation overnight at 37°C, 150 µL supernatant was collected and stored at -20°C until use.

### Proliferation assays

HSPC-NK cells were cultured according to our culture protocol for 35 days. After 35 days, NK cells were labeled with 1 µM CFSE as described previously. After staining, cells were resuspended in differentiation medium at a concentration of 2E+6/mL and plated in duplicate in a 96-well plate. CsA (Biovision Incorporated, Milpitas, CA, USA) or MPA (Sigma-Aldrich, Zwijndrecht, The Netherlands) were added to final concentrations of 0.01 to 1 µg/mL (CsA) or 0.1 to 10 µg/mL (MPA). Half of the medium containing MPA or CsA in the final concentration was refreshed every 2-3 days. After 7 days, cells were harvested. The number of viable target cells was quantified by FCM by gating on forward scatter and side scatter and exclusion of 7-AAD positive cells. Proliferation was analyzed by determining the CFSE dilution within CD56+ cells.

### Statistical analysis

Results from different experiments are described as mean ± standard error of the mean (SEM). Statistical analysis was performed using a one-tailed paired student's t-test, a two-tailed unpaired student's t-test or one-way ANOVA if values had a normal distribution (normality was determined using the Kolmogorov-Smirnov test). For values without normal distribution we used the Wilcoxon matched-pairs signed rank test. Differences were considered to be significant for p values <0.05.

### Results

### SR1 enhances expansion of PB and BM-derived CD34+ HSPCs and improves NK cell differentiation

In this study, we investigated whether NK cells could be generated *in vitro* from PB or BM-derived CD34+ HSPCs. We used the feeder-free culture protocol described in Figure 1A, which was reported previously to generate high numbers of functional NK cells from UCB-derived CD34+ HSPCs [5,6]. However, in our initial cultures using PB or BM derived HSPCs, expansion and differentiation were low, which was associated with high numbers of stroma-like cells observed in culture plates (data not shown). Therefore, we investigated transfer of non-adherent cells to a new culture plate after 3 days of culture. Although this resulted in much lower outgrowth of stromal cell layers in the culture plates, expansion and NK cell differentiation were still low. For that reason, we investigated whether addition of the AhR antagonist SR1 (2 µM), which is known to improve expansion of CD34+ HSPCs and to influence NK cell differentiation, could improve NK cell generation from HSPCs. Importantly, addition of SR1 strongly enhanced expansion of CD34+ cells from both PB-derived HSPCs (Figure 1), as well as BM-derived HSPCs (Figure 2A-B). Mean NK cell yield after 5 weeks of culture was 235 fold (range 115 - 904 fold; n = 10, Figure 1D) for G-CSF mobilized CD34+ cells, and 129 fold (range 33 - 301; n=4, Figure 2B) for BM-derived CD34+ HSPCs. Interestingly, differentiation also improved by addition of SR1 to our cultures, resulting in a purity of 83% ± 9% for G-CSF mobilized CD34+ HSPCs, and 84% ± 18% for BM-derived CD34+ cells. The remaining non-NK cells in the cultures represented mainly CD14+ and/or CD15+ mature monocytic and myelocytic cells (11% ± 6%). A small frequency of CD34+ cells could also be detected at the end of the culture process (0.7% ± 0.4%). Most importantly, the amount of CD3+ T cells in the SR1-based NK cell cultures investigated was very low (0.1% ± 0.1%). Altogether, these data demonstrate that the AhR pathway negatively influences NK cell development, and that by combining SR1 with cytokine mixtures, high numbers of CD56+ NK cells can be generated from G-CSF-mobilized and BM-derived CD34+ HSPCs *ex vivo.* Since G-CSF-mobilized CD34+ HSPCs are the main stem cell source for allo-SCT, we concentrated on these HSPC-NK cells for further investigations.

### SR1 influences expression of transcription factors important for NK cell differentiation and maturation

To gain insight into the molecular processes behind the SR1-enhanced differentiation of CD34+ HSPCs into NK cells, we analyzed the gene expression profile of several transcription factors that are described to be important for NK cell differentiation and maturation [27-31]. To analyze the culture composition, we concomitantly determined the percentage of CD56+ cells in our cultures at different time points (Figure 3A). Next, we compared expression levels of several transcription factors in the presence or absence of SR1 in total cells at these time points in our *ex vivo* culture system (Figure 3B). We observed efficient down regulation of AhRR (i.e. direct target gene of AhR signaling [14]) in the presence of SR1, indicating that a concentration of 2 µM SR1 is sufficient for AhR inhibition. Interestingly, we observed higher expression of thymocyte selection-associated HMG box factor (TOX), which is important in early NK cell differentiation [27], from day 7 onward (Figure 3B-C). This suggests that SR1 increased the number of NK cell precursors, even before the induction of NK cell differentiation was initiated by addition to IL-15 to the culture, and before CD56 acquisition. Expression of ID2, which is important for NK cell maturation [28,29], was increased from day 14 onwards, after addition of IL-15 to the culture (Figure 3B). Eomesodermin (EOMES), another factor important for NK cell maturation, was upregulated from week three onward. Finally, expression of GATA-3 and Ewing's sarcoma-associated transcript 2 (EAT-2; SH2D1B), required to develop cytotoxic functions [30,31], was increased in cells cultured in the presence of SR1 (Figure 3B).

To investigate whether the increased expression levels of these transcription factors reflected a difference between NK cells generated in the presence or absence of SR1, or resulted from the different composition of the cultures, CD56+ NK cells, generated in the presence or absence of SR1, were sorted by FACS from HSPC-NK cultures after 5 weeks. Subsequently, gene expression levels of the previously mentioned transcription factors were determined. We did not observe increased expression of the NK cell differentiation factors TOX, ID2 and EOMES suggesting that SR1 increases the number of NK cell progenitors resulting in more NK cells, but it does not intrinsically change HSPC-NK cells (Figure 3D). In addition, expression levels of GATA-3 and EAT-2 were similar in CD56+ NK cells cultured in the presence of SR1. Expression of GZMB and PERF was slightly higher, but not significantly increased. Furthermore, IFN-γ was not increased in NK cells cultured in the presence of SR1, but these cells were not exposed to target cells prior to mRNA analysis.

Collectively, these data demonstrate that addition of SR1 to our *ex vivo* culture system blocks function of AhR, resulting in the upregulation of several transcription factors that are required for NK cell differentiation and maturation.

### SR1-generated HSPC-NK cells have an activated and mature phenotype

To further elucidate the effect of SR1 on NK cell activation status and function, we investigated the influence of SR1 on the phenotype of our *ex* vivo-generated CD56+ NK cells. After 35 days of culture in the presence of SR1, NK cell phenotype was analyzed using FCM (Figure 4A-D, Figure 2C). SR1-generated NK cells expressed high levels of NKG2A, which indicates transition between stage 3 and stage 4 NK cell progenitors [32,33]. CD16, important for antibody-dependent cell mediated cytotoxicity (ADCC), was expressed on 22.7 ± 2.6% of the CD56+ cells. Furthermore, we found high expression levels of the activating markers DNAM-1, NKG2D, NCRs, and TRAIL. Expression of KIRs was observed in a low percentage of CD56+ cells. However, expression levels were similar as observed for UCB-NK cells generated in our culture system [5,6,32]. Importantly, our NK cells also expressed high levels of CD62L and chemokine (C-X-C motif) receptor 3 (CXCR3), which are involved in homing to the lymphoid organs and trafficking of NK cells towards inflammation *in vivo* [7,34,35]. Interestingly, presence of CD62L, CXCR3, DNAM-1 and TRAIL were significantly higher in CD56+ cells generated in the presence of SR1 (Figure 4C-D). Increased expression of DNAM-1 and TRAIL suggests that NK cells generated in the presence of SR1 are more active as compared to NK cells generated in the absence of SR1. We did not find significant differences in expression of the other molecules in the cultures with SR1 compared to the cultures without SR1 (data not shown). Altogether, these results indicate that SR1-generated HSPC-NK cells consist of a mixture of immature and mature NK cells expressing various activating receptors, similarly to our previously described UCB-derived NK cells [6].

### SR1-generated HSPC-NK cells have efficient IFN-γ production capacity and cytolytic activity against AML and MM cells

Next, we investigated the functional activity of SR1-generated NK cells, and compared them with NK cells generated in the absence of SR1. After 5 weeks of culture, NK cell products were harvested and co-cultured overnight with AML or MM tumor cell lines. Target cell induced IFN-γ production was determined. We found that our SR1-generated HSPC-NK cells have a good IFN-γ producing capacity, which is improved in cells generated in the presence of SR1 (Figure 5A). Subsequently, we compared the killing capacity of NK cells generated in the presence of absence of SR1. Therefore, 1E+4 CD56+ NK cells were co-cultured overnight with different AML cell lines. We observed significantly improved killing of K562 and HL-60 cells, and also a trend towards improved killing of THP-1 cells by SR1-generated NK cells (Figure 5B). To rule out an effect of non-NK cells in the cultures, we repeated the killing experiments using sorted NK cells. Therefore, CD56+ NK cells were sorted from the NK cell products after 5 weeks of culture. Next, NK cells were co-cultured overnight with AML or MM tumor cell lines as described earlier. Importantly, we also observed higher killing of K562, HL-60 and RPMI8226 cells by sorted SR1-generated NK cells (Figure 5C). In addition, granzyme B production was enhanced in SR1-generated HSPC-NK cells as compared to NK cells generated in the absence of SR1 (Figure 5D). These results indicate that addition of SR1 to our culture system not only increases the number of CD56+ cells we can generate, but also enhances the functional activity of the CD56+ NK cells.

To further confirm the functional activity of SR1-generated NK cells, we investigated degranulation of these cells after overnight co-culture with AML or MM tumor cell lines. We found marked degranulation of our SR1-generated NK cells upon co-culture with the different cell lines (Figure 5E-F). Most efficient degranulation was observed against MHC^{neg} K562 cells as well as the MHC-expressing AML cell line HL-60. We also observed marked degranulation upon co-culture with the MM cell lines U266 and RPMI8226. The MM cell line UM9 was the least potent cell line to induce degranulation. Next, we performed cytotoxicity experiments using SR1-generated HSPC-NK cells harvested after 5 weeks of culture. For this, NK cells were co-cultured overnight with different CFSE-labeled AML (K562, HL-60 and THP-1) and MM (UM9, U266 and RPMI8226) cell lines. The next day, specific killing was determined by FCM. We observed very efficient killing of most AML and MM cell lines, even at an E:T ratio of 1:1 (Figure 5G). Subsequently, we investigated whether patient-derived primary AML blasts were susceptible to killing by SR1-induced NK cells. Therefore, we performed cytotoxicity assays with AML blasts from 5 different patients. Importantly, AML blasts could be potently killed within 3 days of co-culture (Figure 5H). We observed some variance in susceptibility between the different patients, but variance between HSPC-NK cell donors was small, indicating that the quality of the SR1-induced NK cell products is consistent.

Taken together, these data demonstrate that SR1-induced HSPC-NK cells, generated from G-CSF-mobilized CD34+ cells, mediate efficient IFN-γ production, degranulation and cytolytic activity against hematological tumor cells. Furthermore, as expected by increased expression of activating markers, SR1 augments IFN-γ production and cytotoxic activity of *ex* vivo-generated HSPC-NK cells.

### HSPC-NK cell proliferation, viability and function is inhibited by mycophenolic acid (MPA) but not by cyclosporin A (CsA)

Increased NK cell numbers after non-myeloablative allo-SCT have been associated with decreased relapse rates, without an increased risk for GVHD [8,9]. Hence, adoptive transfer of *ex* vivo-generated NK cells shortly after allo-SCT is an attractive approach to improve patient outcome. Our therapeutic strategy is to apply HSPC-NK cell adoptive transfer for high-risk patients treated with non-myeloablative allo-SCT (Figure 6A). For this purpose, we want to exploit 15-30E+6 CD34+ cells from the G-CSF-mobilized donor stem cell graft, which represents approximately 5-10% of the total graft, for *ex vivo* NK cell generation. These HSPC-NK cells can be infused as a single infusion, or multiple infusions after allo-SCT, to improve the GVT effect. However, these allo-SCT patients are treated with immunosuppressive drugs to prevent GVHD, so the effect of these drugs on HSPC-NK cell proliferation and viability should be known. For that reason, we investigated the effect of CsA and MPA, which are often used after non-myeloablative allo-SCT, on SR1-induced NK cells. For this, week 5 HSPC-NK cells were cultured for 7 additional days in the presence of absence of therapeutic concentrations of MPA or CsA (Figure 6B-F). After 7 days, we analyzed NK cell proliferation and viability using FCM (Figure 6B-D). We found that proliferation of HSPC-NK cells was already inhibited by MPA in a therapeutic concentration of 0.1 µg/ml. In contrast, CsA, even at a concentration of 1 µg/ml, did not inhibit HSPC-NK cell proliferation (Figure 6B-C). In addition, NK cell viability was not affected by CsA, while MPA induced dose-dependent cell death (Figure 6D). Next, we investigated the effect of MPA and CsA on HSPC-NK cell function. For this, CD56+ NK cells cultured in the presence of MPA or CsA were co-cultured overnight with K562 cells at an E:T ratio of 1:1. As a control, week 6 CD56+ NK cells were used. We observed impaired killing by NK cells cultured in the presence of MPA; however, CsA did not inhibit NK cell-mediated killing (Figure 6E). In addition, we observed impaired IFN-γ production after incubation with MPA, while incubation with CsA even enhanced IFN-γ production (Figure 6F). These data indicate that adoptive transfer of SR1-generated HSPC-NK cells can be applied in patients treated with CsA, but infusion should be postponed until cessation of MPA therapy.

### SR1 is a key ingredient driving NK cell differentiation in a cytokine-based culture method

In earlier studies, we exploited a phased based addition of cytokines in heparin-based basal media (5-7). Heparin is able to bind certain cytokines, reducing their degradation and perhaps presenting them in a more physiological manner. Furthermore, low dose cytokine cocktail containing G-CSF, GM-CSF and IL-6 was being used, which was based on studies using the fetal liver-derived stromal cell line AFT024 [59]. Since we observed that the AhR antagonist is a crucial component for the *ex vivo* expansion of NK cells from BM and mobilized blood CD34+ HSPCs, we investigated whether we could simplify the culture method by leaving out heparine and low dose cytokines G-CSF, GM-SCF and IL-6 that have mainly an effect on myeloid progenitor cells (see Figure 7). Optimization experiments showed that culturing in the presence of 2 µM SR1 for 21 days promotes the expansion of CD56+ NK cells from CD34+ cells enriched from UCB, BM and mobilized blood (Figure 8A-C). Similar NK cell purity and yield was observed with our innovative SR1-based culture method in the absence of low dose cytokine and heparin (Figure 8 versus Figure 1 and 2). The SR1-based NK cell cultures were still devoid of CD3+ T cells and CD19+ B cells and the remaining non-NK cells represented mainly CD14+ monocytic cells (<20%), see Figure 9. These data confirm that antagonism of the AhR pathway strongly promotes NK cell development, and that SR1 is the key ingredient in our innovative cytokine-based culture method for generating high numbers of CD56+ NK cells from G-CSF-mobilized, UCB- and BM-derived CD34+ HSPCs *ex vivo.*

### Discussion

NK cells are the first lymphocyte population recovering after allo-SCT, and have several important functions shortly after allo-SCT [36]. First of all, they are involved in defence against viral infections such as cytomegalovirus (CMV) infections [37,38], which can cause high morbidity shortly after allo-SCT. Furthermore, high NK cell numbers shortly after non-myeloablative and T cell-depleted allo-SCT have been associated with reduced relapse rates without an increased risk of GVHD [8,9], indicating that allogeneic donor NK cells have specific antitumor activity. Nevertheless, it was shown that early engrafting NK cells have decreased cytokine producing capacity [39]. So, in order to further exploit the beneficial effects of NK cells after allo-SCT, adoptive transfer of functional and rapidly maturating NK cells would be an attractive immunotherapeutic strategy. Since donor-derived NK cells will not be rejected post-transplant, multiple NK cells infusions without the need for immunosuppressive chemotherapy to prevent rejection, will be feasible in this setting. However, high numbers of functional NK cells of donor origin are needed to apply this strategy. Since isolation of sufficient numbers of NK cells from donors, without contaminating alloreactive T cells, is challenging; we investigated if high numbers of functional NK cells could be generated from G-CSF-mobilized CD34+ HSPCs, using an *ex vivo* cytokine-based culture protocol. This protocol was developed by our group earlier, and high numbers of pure and functional NK cells with in vivo maturation capacity, can be generated from UCB-derived CD34+ HSPCs using this GMP-compliant protocol [5-7].

However, we found that NK cell expansion and differentiation from G-CSF-mobilized and BM-derived CD34+ cells in the absence of SR1 was very limited, as compared to NK cell expansion and differentiation of UCB-derived CD34+ HSPCs observed in our previously developed culture protocol. Interestingly, we found that addition of the AhR antagonist SR1 to our cultures greatly enhanced NK cell expansion and differentiation. As a result, we can expand G-CSF-mobilized CD34+ cells on average 268-fold using our newly developed SR1-based protocol. In addition, SR1-generated NK cell products are 83% ± 9% pure. The remaining non-NK cells in the cultures represented mainly CD14+ and/or CD15+ mature monocytic and myelocytic cells. Probably due to SR1 addition still some remaining low amount CD34+ cells could be detected. However, contaminating CD3+ T cells were either not detectable or at very low frequency (0.1% ± 0.1%). Furthermore, we found that SR1-NK cells have a similar phenotype as our NK cells previously generated from UCB-derived CD34+ HSPCs, which is a highly active phenotype, characterized by expression of high levels of activating NK cell receptors [5-7]. Interestingly, expression levels of CD62L, CXCR3, DNAM-1 and TRAIL were even higher on NK cells generated in the presence of SR1. CD62L and CXCR3 are involved in homing to lymphoid organs and trafficking towards inflammation *in vivo,* so high expression of these markers can contribute to trafficking of NK cells toward hematological tumor cells in the lymphoid organs [7,34,35], In addition, it was described that CD62L indicates an unique subset of polyfunctional NK cells combining the ability to produce IFN-γ with cytotoxic properties [40]. DNAM-1 and TRAIL are activation markers, and increased expression levels of these markers suggests that SR1-generated NK cells are more active as compared to NK cells generated in the absence of SR1. This was indeed confirmed in functional studies showing that SR1-induced NK cells have increased INF-γ production capacity and an increased capability to kill AML cell lines, as compared to NK cells generated in the absence of SR1. Furthermore, SR1-generated NK cells showed efficient degranulation against AML and MM cell lines. Besides, even at low E:T ratios, these cells efficiently kill hematological tumor cell lines, and most importantly, patient-derived primary AML blasts. To further investigate the applicability of our SR1-NK cell product after allo-SCT, we investigated the effect of the immunosuppressive drugs MPA en CsA, which are commonly used after non-myeloablative allo-SCT, on our SR1-NK cell product. Importantly, we found that therapeutic CsA concentrations do not affect HSPC-NK cell viability, proliferation or function, while exposure to therapeutic levels of MPA did have a negative effect. Exposure to CsA did even enhance IFN-γ production by the HSPC-NK cells. This is in accordance with the effect of CsA and MPA on naturally occurring NK cells described in literature [41,42]. These results provide strong rational to infuse SR1-generated HSPC-NK cells relatively short after allo-SCT, since MPA treatment is generally stopped at day 28 after allo-SCT. CsA, which is usually prescribed for at least six months, will most likely not affect NK cell viability, proliferation or function in vivo upon transfer. Several methods to generate NK cell products from donor origin for immunotherapy have been reported [43-48]. In most studies, NK cells are isolated from aphaeresis products using magnetic cell sorting systems. However, poor recovery and viability is a common problem in these procedures, therefore NK cells require cytokine stimulation and expansion before infusion [2,46-50]. NK cell numbers up to 7.6E+8 have been reported using this method [49]. Nevertheless, large-scale aphaeresis procedures are necessary to obtain this amount of NK cells, and contaminating T cells can potentially cause GVHD. To circumvent these problems, NK cell generation from CD34+ HSPCs is an attractive option. Yoon et al. recently reported safe infusion of NK cells generated using a feeder-free 6-week culture procedure after HLA-mismatched allo-SCT. Using this procedure, an average number of 9.28E+6 NK cells/kg was generated from 2.22E+6 donor CD34+ HSPCs/kg [45]. In our system, the average NK cell expansion from mobilized CD34+ HSPCs was 235 fold (range 115 - 904 fold). Therefore, if we use 10% of the G-CSF-mobilized CD34+ HSPCs isolated from donors for allo-SCT, we will have on average 0.5E+6 CD34+ cells/kg. In case of a 70 kg donor and patient, we will get on average 8E+9 NK cells (> 1E+8 NK cells/kg). So we will be able to infuse large numbers of NK cells from donor origin using our SR-1 based NK cell generation protocol.

In the present study, we showed that the AhR antagonist SR1 greatly enhances NK cell expansion and differentiation. AhR is a ligand-inducible transcription factor which has extensively been studied in the context of its activation by environmental pollutants, such as dioxins and polycyclic aromatic hydrocarbons [51,52]. Until recently, little was known about the physiological role of AhR, but a growing body of evidence shows that AhR has multiple endogenous activators [15], and is involved in several physiological processes [22,53,54]. Examples of endogenous ligands are metabolites of dietary substances [15,55] and tryptophan metabolites like cinnabarinic acid [56], 6-formylindolo[3,2-b]carbazole (FICZ), which can be produces in the skin upon light exposure [57], and the indoleamine 2,3-dioxygenase 1 (IDO1)-metabolite kynurenin [58]. AhR also regulates differentiation of various immune cells like T helper 17 (TH17) cells [22], dendritic cells [17,18], regulatory T cells [19,20] and γδ T cells [21]. Recently, Hughes et al. reported that antagonism of AhR promotes differentiation of immature innate lymphoid cells into NK cells expressing EOMES and TBET [23]. In our SR1-based HSPC-derived NK cell culture, we also observed up-regulation of EOMES, which is involved in NK cell maturation [29], and additionally we found upregulated expression of TOX, ID2, GATA-3 and EAT-2 after AhR inhibition. TOX is important for early NK cell development [27], ID2 is involved in NK cell maturation [28] and GATA-3 and EAT-2 are important for NK cell effector functions [30,31]. Interestingly, expression of TOX markedly decreased in the first week of culture in our culture system in the absence of SR1. In the presence of SR1, TOX expression is more preserved, resulting is significantly higher TOX expression after one week. This suggests that before induction of differentiation by addition of IL-15, early NK cell progenitors are expanded in the presence of SR1, explaining increased NK cell numbers generated in the presence of SR1, IL-15 and IL-2/IL-12. To our knowledge, this is the first report of an applying AhR blocking in an *in vitro* system to generate high numbers of functional NK cells for therapeutic application.

In conclusion, we developed an SR1 and cytokine-based *ex vivo* culture system, which enables us to generate high numbers of very potent NK cells from G-CSF-mobilized CD34+ HSPCs. Addition of SR1 to the culture system induced up-regulation of multiple NK cell-related transcription factors, and resulted in generation of NK cell products with very high cell numbers and purity. These NK cells have an active phenotype and are highly functional *in vitro,* therefore they hold great promise for future adoptive HSPC-NK cell therapy after allo-SCT as well as for adoptive cell therapy in the non-transplant setting against hematological and solid malignancies.

### Example 2. The aryl hydrocarbon receptor antagonist StemRegenin1 combined with IL-15 and IL-12 drives the generation of CD34+ umbilical cord blood HSPC-derived NK cell products with superior purity and functional characteristics.

### Abstract

Adoptive transfer of allogeneic natural killer (NK) cells represents a promising treatment approach against haematological and solid cancers. Here, we report a highly efficient SR1-based culture method for the generation of CD56+NKG2A+ Perforin+EOMES+LFA-1+ NK cell products with high cell number and purity. In this system, CD34⁺ HSPCs from umbilical cord blood are expanded and differentiated into NK cells under stroma-free conditions in the presence of SR1, IL15 and IL12. We demonstrate that this inventive method drives the generation of more pure, more mature and highly functional NK cells. Particularly, these SR1-expanded HSPC-NK cells mediate significant IFNγ production and cytotoxic activity towards tumor targets. Furthermore, HSPC-NK cells actively infiltrate, migrate and mediate killing of ovarian cancer spheroids using an *in vivo*-like model system and mouse xenograft model. These findings demonstrate that SR1/II,15/II,12-expanded HSPC-NK cells efficiently kill hematological tumor cells, destruct tumor spheroids *in vitro* and target intraperitoneal tumors *in vivo,* providing great promise for effective adoptive immunotherapy in cancer patients.

### Introduction

Natural killer (NK) cells have gained significant attention for adoptive cell therapy (ACT) of cancer due to their well-documented antitumor function. Their natural ability to lyse tumor cells without prior sensitization, their pivotal role in antitumor responses and immunesurveillance against cancer, along with the observations that NK cells do not cause graft-versus-host disease (GVHD) while maintaining graft-versus-tumor (GVT) immunity after allogeneic stem cell transplantation (allo-SCT) have all been central to the overall promising application of NK cells for adoptive immunotherapy of cancer. However, effective ACT requires NK cells to be appropriately activated, available in sufficient numbers, have appreciable persistence *in vivo,* home to the tumor site and effectively kill tumor cells upon encounter. Generally, allogeneic NK cell products have been enriched from the peripheral blood (PB) of haplo-identical donors followed by overnight activation with IL-2 or IL-15. However, this cellular product is rather heterogeneous with 25-95% of infused cells being NK cells depending on the used enrichment method, and contains a variable number of monocytes, B cells and potentially alloreactive T cells capable of inducing GVHD [2,3,50,60-62]. Furthermore, this approach yields relatively low NK cell numbers enough for a single dose, but higher and multiple dosing requires further expansion before adoptive transfer [63-65]. For these reasons, development of more homogeneous, scalable and "off-the-shelf' allogeneic NK cell products is preferable for adoptive immunotherapy.

Alternatively, NK cells can be efficiently generated *ex vivo* from hematopoietic stem and progenitor cells (HSPC) or induced pluripotent stem cells (iPSC) [5-7,45,66,67]. Previously, we have reported good manufacturing practice (GMP)-compliant, cytokine-based culture protocols for the *ex vivo* generation of highly active NK cells from CD34+ HSPCs isolated from various stem cell sources [5, 7, 66]. By applying the aryl hydrocarbon (AhR) antagonist StemRegenin1 (SR1), and the combination of IL-15 and IL-12 we have shown that highly active CD56+ NK cells can be generated with potent cytolytic activity towards hematological tumor cells *in vitro* [66] as well as anti-leukemic effects *in vivo* following IV administration [7]. In the present study, we investigated the preclinical efficacy of *ex vivo* generated, highly functional CD56+Perforin+ HSPC-NK cells generated by an optimized SR1/IL15/IL12 based culture protocol in clinically relevant tumor models.

### Materials and Methods

### Cell lines

Tumor cell lines SKRC52, SKRC17, SKOV-3 and IGROV1 were cultured in Roswell Park Memorial Institute medium (RPMI 1640; Invitrogen, Carlsbad, CA) medium with 10% Fetal Calf Serum (FCS; Integro, Zaandam, The Netherlands). The OVCAR-3 cell line was cultured in RPMI 1640 medium with 20% FCS and 1 µg/ml insulin. K562 cells were cultured in Iscove's Dulbecco's medium (IMDM; Invitrogen, Carlsbad, CA) containing 10% FCS. SKOV-3-GFP-luc cells were generated by stable transduction of parental cells with lentiviral particles LVP20 encoding the reporter genes green fluorescent protein (GFP) and luciferase (luc) under control of the CMV promoter (GenTarget, San Diego). Transduced cells were cloned and an optimal SKOV-3-GFPluc clone for *in vitro* and *in vivo* experiments was selected based on GFP expression, luciferase activity, and comparable susceptibility to HSPC-NK killing as the parental cells.

### Patient-derived ovarian cancer (OC) cells

Patient material from stage III and IV OC patients was obtained before primary treatment in the Radboud University Medical Center (RUMC) after written informed consent. Fresh ascites was filtered using a 100 µm filter, centrifuged and resuspended in PBS/0.5% BSA. Subsequently, mononuclear cells were isolated using a Ficoll-Hypaque (1.077 g/ml; GE Healthcare, Uppsala, Sweden) density gradient centrifugation. Isolated cells were washed and cultured in RPMI 1640 medium with 10% FCS. After 24 hours, non-adherent cells were removed by washing with PBS. After 3 days, medium was refreshed. At day 6-8, cells were trypsinized and analyzed for percentage tumor cells by flow cytometry (FCM). After reaching 90% confluency, OC cell layers were used for either tumor spheroid culture or directly for co-culture experiments.

### Multicellular tumor spheroids

OC tumor spheroids were generated by seeding 3×10⁴ cells/well in a volume of 200 µl/well of culture medium in 96-well plates coated with 1% agarose in DMEM F12 medium as described previously by Giannattasio et al. [68] with minor adaptations. 3D tumor spheroids were used for functional assays upon reaching a solid state after 72 h after initial seeding.

### HSPC-NK cell generation

Umbilical cord blood (UCB) units were collected at Caesarean sections after full term pregnancy and obtained informed consent of the mother. CD34+ HSPCs were isolated from mononuclear cells after Ficoll-Hypaque density gradient centrifugation and CD34-positive immunomagnetic bead selection (Miltenyi Biotec, Bergisch Gladbach, Germany). After isolation, CD34⁺ HSPCs were either cryopreserved or directly used for NK cell generation. Cultures were performed over 5-6 weeks in 6-well tissue culture plates (Corning Inc., NY) or Vuelite^{™} AC-bags (Cellgenix, Freiburg, Germany), using CellGro DC medium (Cellgenix) or NK MACS medium (Miltenyi) supplemented with 10% and 2% human serum (Sanquin Bloodbank, Nijmegen, The Netherlands) during the expansion and the differentiation phase, respectively. CD34+ cells were first cultured for 9 or 10 days with 2 µM SR1 (Cellagen Technology, San Diego, CA), 25 ng/mL IL-7 (Immunotools), 25 ng/mL SCF (Immunotools), 25 ng/mL Flt3L (Immunotools) and 25 ng/mL TPO (CellGenix). From day 9 or 10 until day 14 or 15, TPO was replaced by 50 ng/mL IL-15 (Miltenyi). After day 14 or 15, cells were cultured in differentiation medium consisting of 20 ng/mL IL-7, 20 ng/mL SCF, 50 ng/mL IL-15, and 0.2 ng/ml IL-12 (Miltenyi). SR1 was added till day 21. Total cell number and CD56 acquisition were analyzed twice a week by flow cytometry, and medium was refreshed every 2 to 4 days to keep cell density between 1.5E+6 and 2.5E+6 cells/ml. Cultures were maintained at 37°C, in 95% humidity, and 5% CO2. HSPC-NK cell products were used in experiments after 5 to 6 weeks of culture with > 80% CD56⁺ cells.

### Flow cytometry (FCM)

Cell numbers and expression of cell-surface markers were determined by FCM. Antihuman CD45-ECD (J.33; Beckman Coulter, Woerden, The Netherlands) and anti-CD56-PC7 (HCD56; BioLegend, San Diego, CA) antibodies were used to follow cell number and NK cell differentiation during culture using the Coulter FC500 flow cytometer (Beckman Coulter). The population of viable cells was determined by exclusion of 7-AAD-positive cells (Sigma, St. Louis, MO). For phenotypical analysis, cells were incubated with panels of antibodies in the FCM buffer (PBS/0.5% BSA) for 30 min at 4°C. After washing, cells were resuspended in the FCM buffer and analyzed.

### FCMbased degranulation and IFNyproduction assay

HSPC-NK cell degranulation and IFNγ production were determined upon stimulation with K562 cells at an E:T ratio of 1:1. Anti-CD107a (H4A3; BD Biosciences) was added at a 1:100 dilution at the start of the co-culture, and brefeldin A (1 ng/mL, BD Biosciences) was added 1 h later. Cells were collected the following day, stained for cell surface markers, and then treated with fixation/permeabilization buffer (eBioscience) and stained for intracellular Perforin and IFNγ. Flow cytometric analysis was performed with exclusion of dead cells with Fixable Viability Dye (eBioSciences) and using non-stimulated cells as a control.

### FCM-based cytotoxicity and infiltration assays

In monolayer cytotoxicity assays, tumor cells were first seeded at a concentration of 3E+4 in flat bottom 96-well plates in triplicate for each test condition. The following day, HSPC-NK cells were labeled with 1 µM carboxyfluorescein diacetate succinimidyl ester (CFSE; Molecular Probes; Invitrogen, Eugene, OR) and were added at 3 different effector-target (E:T) ratio's (1:1, 3:1 and 10:1). For cytotoxicity assays with SKOV-3-GFPluc cells, HSPC-NK cells were added without CFSE staining. After 24 hours, co-culture supernatants were harvested and stored at -20°C until use for ELISA. Subsequently, cells were detached using trypsine (for solid tumor cell lines), resuspended and collected in micronic tubes. The life/dead marker 7AAD (1/1000 final dilution) was added to the cells and absolute numbers of viable targets present in each well were determined by FCM (FC500, Beckman Coulter). Specific lysis of tumor cells by NK cells was calculated with the following formula: 100% - [(absolute number of viable target cells after co-culture with NK cells/absolute number of viable target cells cultured in medium) × 100%].

In cytotoxicity assays with OC spheroids, day three multicellular spheres were used. HSPC-NK cells were added to the tumor spheroids at 3 different E:T ratio's. After 24 hours of co-culture, supernatant was collected for ELISA. Subsequently, spheroids were washed, trypsinized and viable 7AAD-negative tumor cells were counted using the FC500 flow cytometer.

The production capacity of Granzyme-B and Interferon-γ by HSPC-NK cells were evaluated by ELISA according to the manufacturer's instructions (IFN-γ; Pierce Endogen, Rockford, USA and Granzyme-B; Mabtech, Sweden).

For the FCM-based infiltration assay, we performed the OC spheroid-NK cell co-culture assay as described above. After different incubation time points, tumor spheroids were harvested with a cut 1000 µl-tip, pooled and separated from the supernatant containing the surrounding NK cells and residual target cells by mild centrifugation. Following two washing steps in PBS/2%FCS, tumor spheroids were dissociated using trypsin. The resulting single cell suspension was washed again and stained with CD56 and NKG2A antibodies, and the life/dead marker 7AAD. The percentage of viable CD56+NKG2A+ NK cells was determined by FCM (FC500 flow cytometer).

### Confocal microscopic imaging of HSPC-NK cell invasion in tumor spheroids

Co-culture of OC spheroids and HSPC-NK cells was performed as described above. For confocal microscopic experiments, SKOV-3-GFPluc spheroids were co-cultured with CD56APC-labeled NK cells. At 4, 18 and 24 hours of co-culture, spheres were collected, washed mildly with PBS, and placed in an ibidi 1µ-slide 8-wells plate (ibidi GmbH) in RPMI without phenol red. Imaging was performed with the Leica TCS SP5 microscope. Images were processed with Fiji imageJ software.

### HSPC-NK cell adoptive transfer, intraperitoneal SKOV-3 model, and bioluminescence imaging

NOD/SCID/IL2Rg^{null} (NSG) mice originally purchased from Jackson laboratories were housed and bred in the RUMC Central Animal Laboratory. All animal experiments were approved by the Animal Experimental Committee of the RUMC and conducted in accordance with institutional and national guidelines under the university permit number 10300. A preclinical OC xenograft model was established using SKOV-3 cells previously engineered to express the GFP and luciferase reporter genes (SKOV-3-GFPLuc). Efficient OC engraftment in 6-12 weeks old NSG mice was achieved using 2E+5 cells SKOV-3-GFPLuc cells injected intraperitoneally (IP). Tumor load was monitored by bioluminescence imaging (BLI) as previously described (7). In brief, mice were injected i.p. with D-luciferin (150 mg/kg) and after 10 min BLI images were collected using the IVIS imager with the LivingImage processing software. Regions of interest (ROIs) were drawn around the abdominal area of the mice, and measurements were automatically generated as integrated flux of photons (photons/s).

In HSPC-NK cell adoptive transfer studies, SKOV-3 injected mice were equally divided into two groups (no treatment versus IP NK treatment groups) after the first BLI at day 3. After 4 and 11 days, mice received two IP NK cell infusions (12×10⁶ cells/mouse/infusion). Recombinant human IL-15 (Miltenyi Biotech) was administrated subcutaneously at a dose of 1 µg (5,000 units) per mouse every 2-3 d, starting the day of HSPC-NK cell infusion till day 21. BLI images were collected weekly till day 63.

### Statistical analysis

Data analysis was conducted by Prism software (GraphPad, version 5.03 for Windows). The survival probability was estimated by Kaplan-Meier methods. Two-way ANOVA, or Student t-test was used to calculate statistically significant differences between groups. A P-value of <0.05 was considered statistically significant.

### Results

### SR1-generated HSPC-NK cell products from UCB units

Recently, we reported that inhibition of AhR using SR1 improved NK cell generation from CD34+ HSPCs, by enhancing the expression of several transcription factors involved in early NK cell development [66]. In addition, we reported that combining IL-15 with IL-12 drives the differentiation of more mature and highly functional HPC-NK cells, which display potent alloreactivity towards haematological cancer cells [68]. In the present study, we combined SR1, IL-15 and IL-12 to generate HPC-NK cells and test their alloreactivity potential against solid tumor cells. As illustrated in Figure 10a, our inventive SR1/IL15/IL12-based method resulted in >1,000 fold expansion of HSPCs and differentiation into >80% CD45+CD56+CD3- lymphoid cells (Figure 10a). After harvesting and washing, the mean fold expansion of 7 HSPC-NK cell products was 1,160-fold (range 845-1,904). Mean percentage of CD56+ cells within the total CD45+ nucleated cell fraction was 95% (range 91-99%; n=7). Mean percentage of NKG2A+ of CD56+ cells was 78% (range 64-97%; n=7). Mean CD56+NKG2A+ NK cell yield was 884E+6 cells from 1E+6 CD34+ HSPCs (range 697-1,240E+6; n=7). Among CD56⁺ cells, conventional NK cells can be identified by Perforin, NKG2A and EOMES expression (Figure 10b). Remaining CD56+ ILC3 cells can be identified by RORyt expression (data not shown). Non-CD56+ cells present in the final product were represented by <10% CD14+ myeloid cells, and <0.5% CD19+ B cells (Figure 10b). Contaminating T cells were virtually absent (< 0.05%). The CD56+Perforin+ NK cells displayed high expression level of activating receptors, homing receptors and adhesion molecules (Figure 10c). Furthermore, these SR1/II,15/II,12-expanded HSPC-NK cells demonstrated high cytolytic activity and IFN-γ production capacity against K562 cells at low E:T ratios (Figure 10d). Potent NK cell activation and reactivity was further confirmed at the single cell level with induction of significant proportions of degranulating CD107a+ and IFNγ+ NK cells upon short term stimulation (Figure 10e). These data demonstrate our inventive SR1/IL15/IL12-based method consistently generates highly functional CD56+Perforin+ HSPC-NK cell product with very high purity.

### Upscaling of SRI-expanded HSPC-NK cell products

Next, we translated our SR1/II,15/II,12-based method into a GMP-compliant, closed system bioprocess in Vuelife^{™} AC culture bags, and compared this with research scale culturing in 6-well plates. For this, we expanded CD34+ UCB cells for 5 weeks in the presence of SR1 using our inventive *ex vivo* culture protocol depicted in Figure 7 using NK MACS medium from Miltenyi. The total HSPC-NK cell expansion after 5 weeks of culture was >2,500 fold in the static bag, which was similar to the plate-cultured cells (Figure 11a). Moreover, the percentage CD56+ cells at 5 weeks of culture was similar between the static bag and plates, which was 95% (Figure 11b). Flow cytometric analysis of the bag-cultured HSPC-NK cell product demonstrated that the SR1/IL15/IL12-expanded final product comprises of >80% CD56⁺NKG2A⁺LFA-1⁺ NK cells with high expression of NKG2D, DNAM-1, CXCR3 and TRAIL (Figure 11c). Furthermore, CD56+Perforin+ HSPC-NK cells generated in the presence of SR1, IL15 and IL-12 display high degranulating activity and mediate a superior IFN-γ response at the single cell level (Figure 12). These data demonstrate that our inventive SR1/IL15/IL12-based method efficiently generates HSPC-NK cell products for adoptive immunotherapy during 5 weeks of culture in static bags.

### SR1-expanded HSPC-NK cells efficiently kill malignant cells

To explore the cytotoxic potential of SR1-expanded HSPC-NK cells against tumor cells, we first performed flow cytoemtry-based cytotoxicity assays with panels of tumor cell lines including frequently used renal cell carcinoma (RCC) and ovarian carcinoma (OC) cell lines (SKRC17, SKRC52, SKOV-3, IGROV1, OVCAR-3) as well as primary OC cells cultured from patient's ascites. The NK-sensitive K562 cells were included as control. Functional analysis demonstrated that SR1-expanded HSPC-NK cells mediate potent cytolytic activity against all solid tumor cell lines and patient-derived OC mono-layers tested (Figure 13 and 14a). At a low E:T ratio of 1:1 between 30% to 90% killing was observed. This increased up to almost 100% at higher E:T ratios (Figure 13 and 14a). These data demonstrate that HSPC-NK cells by our inventive SR1/IL15/IL12-based method are very potent killers of hematological and solid tumor cells.

### SR1-expanded HSPC-NK cells effectively infiltrate and destroy tumor spheroids

Next, we tested the potency of SR1-expanded HSPC-NK cells to infiltrate and kill malignant cells in clinically relevant three-dimensional tumor spheroids. Microscopic evaluation and flow cytometry-based tumor cell quantification demonstrated that HSPC-NK cells target and destroy SKOV-3 spheroids as well as patient-derived OC spheroids after 24 hour co-culture (Figure 14b-d). Furthermore, flow cytometry analysis and live-imaging confocal microscopy demonstrated that SR1-expanded HSPC-NK cells efficiently infiltrate, migrate and kill OC cells in 3D SKOV-3 tumor spheroids (Figure 15). Interestingly, infiltrating CD56+ HSPC-NK cells were observed in the outer third of the 500µ diameter sphere after 4h of co-culture (Figure 15b). Thereafter, CD56+ HPSC-NK cells reached the core of the sphere after 24h of co-culture (Figure 15b). These findings demonstrate highly active HSPC-NK cells potently infiltrate and destroy *in vivo-*like multi-cellular tumor spheroids.

### SR1-expanded HSPC-NK cells efficiently target SKOV-3 OC tumors in vivo

To study the *in vivo* anti-tumor potential of SR1-expanded HSPC-NK cells, adoptive transfer studies were designed in adult NSG mice injected IP with SKOV-3 cells, followed by two HSPC-NK cell infusions, supportive rhIL-15 injections, and weekly bioluminescence measurements (Figure 16a). Importantly, progression of SKOV-3 tumors was significantly reduced by treatment with SR1-expanded HSPC-NK cells (Figure 16b). Furthermore, survival of HSPC-NK cell treated mice was significantly prolonged after SKOV-3 tumor injection. These data demonstrate that infusion of SR1-expanded HSPC-NK cells results in a significant anti-tumor effect in tumor-bearing hosts and provide the rationale to pursue clinical trials using adoptive transfer of HSPC-NK cells in OC patients.

### Conclusion and Discussion

In the past decade, adoptive transfer of allogeneic NK cells gained much attention as a promising adjuvant treatment approach against cancer. Thereby, a great number of reports described new methodologies to achieve higher NK cell numbers and activation. Particularly, expansion and differentiation of HSPCs allows generation of clinically applicable NK cell products with high purity and functionality. Nevertheless, we and others have observed that NK cells generated under stroma-free culture conditions using heparin, IL-15 and IL-2 have low surface expression of CD16 and only a subset (5-10%) expresses KIRs (5-7,69), raising the question about NK cell potency and antitumor reactivity following adoptive transfer. Recently, we demonstrated that more mature and highly functional HSPC-NK cells can be generated under stroma-free conditions by replacement of IL-2 for IL-12 (68). Interestingly, we showed that IL-12 particularly favours the generation of CD16 and KIR-expressing NK cells, resulting in the rapid development of hyper-responsive CD16⁺KIR⁺NK cells following adoptive transfer. In addition, we also reported recently that inhibition of AhR using SR1 improved NK cell generation from CD34+ HSPCs, by enhancing the expression of several transcription factors involved in early NK cell development [66]. In the present study, we combined SR1, IL-15 and IL-12 to generate HSPC-NK cells and tested their cytotoxic potential against solid tumor cells. We demonstrate that our inventive SR1/IL15/IL12-based method results in consistent, clinically applicable *ex vivo* production of highly active HSPC-NK cells. In addition, we demonstrate that the SR1, IL15 and IL12 combination not only improves NK production yields and purity, but also functionality is superior as SR1/II,15/II,2-expanded HSPC-NK cells mediate higher IFNγ production and cytotoxic activity towards tumor targets. Furthermore, these next generation HSPC-NK cells actively infiltrate into and destruct tumor spheroids *in vitro* and *in vivo,* providing great promise for effective adoptive immunotherapy in cancer patients.

Previous methods to generate NK cell products from CD34+ HSPCs have been reported (5,6,69,70). Spanholtz et al. reported a heparin-based culture method for CD34+ UCB cells in a medium comprising heparin, SCF, Flt3L, IL-7, TPO, IL-15, IL-2 and a cocktail of low-dose cytokines (5,6,69). Using this method 3-log expansion of HSPC-NK cells with a purity of >80% of CD56+ cells can be obtained in a bioreactor (6). However, next to relative low CD16 and KIR positivity (6), CD107a-based degranulation assays using K562 as target cells showed a mean frequency of CD107a⁺ degranulating NK cells of 28% ± 5% and IFNγ secreting NK cells of 2.5% ± 1% (n=10), for these NK cell products generated with this heparin/II,15/II,2 based protocol. In contrast, NK cells generated with our new inventive SR1/IL15/IL12-based protocol contained not only higher purity (i.e. >90%) of total CD56+ cells and >80% conventional CD56+Perforin+ NK cells, but also more CD56+Perforin+ NK cells (>10%) capable of mediating an IFNγ response towards K562 target cells.

Previously, we showed that the AhR antagonist SR1 greatly enhances NK cell expansion and differentiation (66). AhR is a ligand-inducible transcription factor which has extensively been studied in the context of its activation by environmental pollutants, such as dioxins and polycyclic aromatic hydrocarbons [51,52]. Until recently, little was known about the physiological role of AhR, but a growing body of evidence shows that AhR has multiple endogenous activators [15], and is involved in several physiological processes including NK cell development [22,53,54]. Recently, Hughes et al. reported that antagonism of AhR promotes differentiation of immature innate lymphoid cells into NK cells expressing EOMES and TBET [23]. In our SR1-based HSPC-derived NK cell culture, we also observed up-regulation of EOMES, which is involved in NK cell maturation [29], and additionally we found upregulated expression of TOX, ID2, GATA-3 and EAT-2 after AhR inhibition. TOX is important for early NK cell development [27], ID2 is involved in NK cell maturation [28] and GATA-3 and EAT-2 are important for NK cell effector functions [30,31]. Interestingly, expression of TOX markedly decreased in the first week of culture in our culture system in the absence of SR1 (66). In the presence of SR1, TOX expression is more preserved, resulting is significantly higher TOX expression after one week. This suggests that before induction of differentiation by addition of IL-15 and IL-12, early NK cell progenitors are expanded in the presence of SR1, explaining increased numbers of conventional CD56+Perforin+NKG2A+LFA-1 + NK cells, and less CD56+ ILC3 cells, generated in the presence of SR1, IL-15 and IL-12. In this regard, Ambrosini et al. very recently reported a culture protocol for CD34+ UCB cells in a medium comprising SCF, Flt3L, IL-7, IL-15, IL-21 in the presence or absence of IL-1β (70). They showed that the addition of IL-1β at the beginning of the culture, i.e. at early stages of differentiation, also inhibits the proliferation of LFA-1-CD161+CD56+ ILC3 cells, while favoring the generation of mature NK cells expressing LFA-1 (CD11a), NKG2A/CD94, KIR, NKp46 and perforin. Although they showed that after 25 days of culture with IL-1β more CD56+ NK cells express CD161, LFA1, NKG2A, NKp46, EOMES and perforin (i.e. conventional NK cells or ILC1 cells) the overall percentage is only <20% in their culture protocol. Furthermore, Ambrosini et al. does not report total CD56+Perforin+ NK cell yields achievable with their IL-1β-based protocol (70). In sharp contrast, our inventive SR1/IL15/IL12 based protocol yields purities of mature CD56+NKG2A+NKp46+Perforin+EOMES+LFA1+ NK cells of >80%. Furthermore, we obtain with our SR1/IL15/IL12-based protocol more than 3-log NK cell expansion resulting in producing at least 1E+9 conventional CD56+Perforin+ NK cells with a high maturation and activation status from one single UCB unit containing >2E+6 CD34+ cells.

In conclusion, we demonstrate that our inventive and scalable SR1/II,15/II,12-based method consistently drives the generation of more pure, more mature and highly functional HSPC-NK cell products with enhanced cytolytic activity and IFNγ production towards tumor cells. Furthermore, we demonstrated that these SR1-expanded HSPC-NK cells actively infiltrate, migrate and mediate killing of solid tumor spheroids in an *in vivo-*like 3D model system and mouse xenograft model. Collectively, our findings demonstrate that SR1/II,15/II,12-expanded HSPC-NK cells efficiently kill hematological tumor cells, destruct tumor spheroids *in vitro* and target intraperitoneal tumors *in vivo,* providing great promise for effective adoptive immunotherapy in cancer patients.

### REFERENCE LIST

1. Ljunggren HG and KJ Malmberg. (2007). Prospects for the use of NK cells in immunotherapy of human cancer. Nat Rev Immunol 7:329-39.
2. Miller JS, Y Soignier, A Panoskaltsis-Mortari, SA McNearney, GH Yun, SK Fautsch, D McKenna, C Le, TE Defor, LJ Burns, PJ Orchard, BR Blazar, JE Wagner, A Slungaard, DJ Weisdorf, IJ Okazaki and PB McGlave. (2005). Successful adoptive transfer and in vivo expansion of human haploidentical NK cells in patients with cancer. Blood 105:3051-7.
3. Curti A, L Ruggeri, A D'Addio, A Bontadini, E Dan, MR Motta, S Trabanelli, V Giudice, E Urbani, G Martinelli, S Paolini, F Fruet, A Isidori, S Parisi, G Bandini, M Baccarani, A Velardi and RM Lemoli. (2011). Successful transfer of alloreactive haploidentical KIR ligand-mismatched natural killer cells after infusion in elderly high risk acute myeloid leukemia patients. Blood 118:3273-9.
4. Rubnitz JE, H Inaba, RC Ribeiro, S Pounds, B Rooney, T Bell, CH Pui and W Leung. (2010). NKAML: a pilot study to determine the safety and feasibility of haploidentical natural killer cell transplantation in childhood acute myeloid leukemia. J Clin Oncol 28:955-9.
5. Spanholtz J, F Preijers, M Tordoir, C Trilsbeek, J Paardekooper, T de Witte, N Schaap and H Dolstra. (2011). Clinical-grade generation of active NK cells from cord blood hematopoietic progenitor cells for immunotherapy using a closed-system culture process. PLoS One 6:e20740.
6. Spanholtz J, M Tordoir, D Eissens, F Preijers, A van der Meer, I Joosten, N Schaap, TM de Witte and H Dolstra. (2010). High log-scale expansion of functional human natural killer cells from umbilical cord blood CD34-positive cells for adoptive cancer immunotherapy. PLoS One 5:e9221.
7. Cany J, AB van der Waart, M Tordoir, GM Franssen, BN Hangalapura, J de Vries, O Boerman, N Schaap, R van der Voort, J Spanholtz and H Dolstra. (2013). Natural killer cells generated from cord blood hematopoietic progenitor cells efficiently target bone marrow-residing human leukemia cells in NOD/SCID/IL2Rg(null) mice. PLoS One 8:e64384.
8. Baron F, EW Petersdorf, T Gooley, BM Sandmaier, M Malkki, TR Chauncey, DG Maloney and R Storb. (2009). What is the role for donor natural killer cells after nonmyeloablative conditioning? Biol Blood Marrow Transplant 15:580-8.
9. Savani BN, S Mielke, S Adams, M Uribe, K Rezvani, AS Yong, J Zeilah, R Kurlander, R Srinivasan, R Childs, N Hensel and AJ Barrett. (2007). Rapid natural killer cell recovery determines outcome after T-cell-depleted HLA-identical stem cell transplantation in patients with myeloid leukemias but not with acute lymphoblastic leukemia. Leukemia 21:2145-52.
10. Tanaka M, S Kobayashi, A Numata, T Tachibana, H Takasaki, A Maruta, Y Ishigatsubo and H Kanamori. (2012). The impact of the dose of natural killer cells in the graft on severe acute graft-versus-host disease after unrelated bone marrow transplantation. Leuk Res 36:699-703.
11. Symons HJ, MS Leffell, ND Rossiter, M Zahurak, RJ Jones and EJ Fuchs. (2010). Improved survival with inhibitory killer immunoglobulin receptor (KIR) gene mismatches and KIR haplotype B donors after nonmyeloablative, HLA-haploidentical bone marrow transplantation. Biol Blood Marrow Transplant 16:533-42.
12. Cooley S, E Trachtenberg, TL Bergemann, K Saeteum, J Klein, CT Le, SG Marsh, LA Guethlein, P Parham, JS Miller and DJ Weisdorf. (2009). Donors with group B KIR haplotypes improve relapse-free survival after unrelated hematopoietic cell transplantation for acute myelogenous leukemia. Blood 113:726-32.
13. Kroger N, T Zabelina, J Berger, H Duske, E Klyuchnikov, T Binder, T Stubig, Y Hilde-brandt, D Atanackovic, H Alchalby, F Ayuk, AR Zander, U Bacher and T Eiermann. (2011). Donor KIR haplotype B improves progression-free and overall survival after allogeneic hematopoietic stem cell transplantation for multiple myeloma. Leukemia 25:1657-61.
14. Boitano AE, J Wang, R Romeo, LC Bouchez, AE Parker, SE Sutton, JR Walker, CA Flaveny, GH Perdew, MS Denison, PG Schultz and MP Cooke. (2010). Aryl hydrocarbon receptor antagonists promote the expansion of human hematopoietic stem cells. Science 329:1345-8.
15. Nguyen LP and CA Bradfield. (2008). The search for endogenous activators of the aryl hydrocarbon receptor. Chem Res Toxicol 21:102-16.
16. Mimura J and Y Fujii-Kuriyama. (2003). Functional role of AhR in the expression of toxic effects by TCDD. Biochim Biophys Acta 1619:263-8.
17. Liu H, I Ramachandran and DI Gabrilovich. (2014). Regulation of plasmacytoid dendritic cell development in mice by aryl hydrocarbon receptor. Immunol Cell Biol 92:200-3.
18. Thordardottir S, BN Hangalapura, T Hutten, M Cossu, J Spanholtz, N Schaap, TR Radstake, RV Voort and H Dolstra. (2014). The Aryl Hydrocarbon Receptor Antagonist StemRegenin 1 Promotes Human Plasmacytoid and Myeloid Dendritic Cell Development from CD34 Hematopoietic Progenitor Cells. Stem Cells Dev.
19. Gandhi R, D Kumar, EJ Burns, M Nadeau, B Dake, A Laroni, D Kozoriz, HL Weiner and FJ Quintana. (2010). Activation of the aryl hydrocarbon receptor induces human type 1 regulatory T cell-like and Foxp3(+) regulatory T cells. Nat Immunol 11:846-53.
20. Hauben E, S Gregori, E Draghici, B Migliavacca, S Olivieri, M Woisetschlager and MG Roncarolo. (2008). Activation of the aryl hydrocarbon receptor promotes allograft-specific tolerance through direct and dendritic cell-mediated effects on regulatory T cells. Blood 112:1214-22.
21. Kadow S, B Jux, SP Zahner, B Wingerath, S Chmill, BE Clausen, J Hengstler and C Esser. (2011). Aryl hydrocarbon receptor is critical for homeostasis of invariant gammadelta T cells in the murine epidermis. J Immunol 187:3104-10.
22. Veldhoen M, K Hirota, AM Westendorf, J Buer, L Dumoutier, JC Renauld and B Stockinger. (2008). The aryl hydrocarbon receptor links TH17-cell-mediated autoimmunity to environmental toxins. Nature 453:106-9.
23. Hughes T, EL Briercheck, AG Freud, R Trotta, S McClory, SD Scoville, K Keller, Y Deng, J Cole, N Harrison, C Mao, J Zhang, DM Benson, J Yu and MA Caligiuri. (2014). The Transcription Factor AHR Prevents the Differentiation of a Stage 3 Innate Lymphoid Cell Subset to Natural Killer Cells. Cell Rep 8:150-62.
24. Overes IM, B de Rijke, A van Horssen-Zoetbrood, H Fredrix, AO de Graaf, JH Jansen, JH van Krieken, RA Raymakers, R van der Voort, TM de Witte and H Dolstra. (2008). Expression of P2X5 in lymphoid malignancies results in LRH-1-specific cytotoxic T-cell-mediated lysis. Br J Haematol 141:799-807.
25. Jedema I, NM van der Werff, RM Barge, R Willemze and JH Falkenburg. (2004). New CFSE-based assay to determine susceptibility to lysis by cytotoxic T cells of leukemic precursor cells within a heterogeneous target cell population. Blood 103:2677-82.
26. Norde WJ, IM Overes, F Maas, H Fredrix, JC Vos, MG Kester, R van der Voort, I Jedema, JH Falkenburg, AV Schattenberg, TM de Witte and H Dolstra. (2009). Myeloid leukemic progenitor cells can be specifically targeted by minor histocompatibility antigen LRH-1-reactive cytotoxic T cells. Blood 113:2312-23.
27. Aliahmad P, B de la Torre and J Kaye. (2010). Shared dependence on the DNA-binding factor TOX for the development of lymphoid tissue-inducer cell and NK cell lineages. Nat Immunol 11:945-52.
28. Boos MD, Y Yokota, G Eberl and BL Kee. (2007). Mature natural killer cell and lymphoid tissue-inducing cell development requires Id2-mediated suppression of E protein activity. J Exp Med 204:1119-30.
29. Gordon SM, J Chaix, LJ Rupp, J Wu, S Madera, JC Sun, T Lindsten and SL Reiner. (2012). The transcription factors T-bet and Eomes control key checkpoints of natural killer cell maturation. Immunity 36:55-67.
30. Samson SI, O Richard, M Tavian, T Ranson, CA Vosshenrich, F Colucci, J Buer, F Grosveld, I Godin and JP Di Santo. (2003). GATA-3 promotes maturation, IFN-gamma production, and liver-specific homing of NK cells. Immunity 19:701-11.
31. Perez-Quintero LA, R Roncagalli, H Guo, S Latour, D Davidson and A Veillette. (2014). EAT-2, a SAP-like adaptor, controls NK cell activation through phospholipase Cgamma, Ca++, and Erk, leading to granule polarization. J Exp Med 211:727-42.
32. Lehmann D, J Spanholtz, M Osl, M Tordoir, K Lipnik, M Bilban, B Schlechta, H Dolstra and E Hofer. (2012). Ex vivo generated natural killer cells acquire typical natural killer receptors and display a cytotoxic gene expression profile similar to peripheral blood natural killer cells. Stem Cells Dev 21:2926-38.
33. Grzywacz B, N Kataria, M Sikora, RA Oostendorp, EA Dzierzak, BR Blazar, JS Miller and MR Verneris. (2006). Coordinated acquisition of inhibitory and activating receptors and functional properties by developing human natural killer cells. Blood 108:3824-33.
34. Beider K, A Nagler, O Wald, S Franitza, M Dagan-Berger, H Wald, H Giladi, S Brocke, J Hanna, O Mandelboim, M Darash-Yahana, E Galun and A Peled. (2003). Involvement of CXCR4 and IL-2 in the homing and retention of human NK and NK T cells to the bone marrow and spleen of NOD/SCID mice. Blood 102:1951-8.
35. Chen S, H Kawashima, JB Lowe, LL Lanier and M Fukuda. (2005). Suppression of tumor formation in lymph nodes by L-selectin-mediated natural killer cell recruitment. J Exp Med 202:1679-89.
36. Storek J, MA Dawson, B Storer, T Stevens-Ayers, DG Maloney, KA Marr, RP Witherspoon, W Bensinger, ME Flowers, P Martin, R Storb, FR Appelbaum and M Boeckh. (2001). Immune reconstitution after allogeneic marrow transplantation compared with blood stem cell transplantation. Blood 97:3380-9.
37. Brown MG, AO Dokun, JW Heusel, HR Smith, DL Beckman, EA Blattenberger, CE Dubbelde, LR Stone, AA Scalzo and WM Yokoyama. (2001). Vital involvement of a natural killer cell activation receptor in resistance to viral infection. Science 292:934-7.
38. Foley B, S Cooley, MR Verneris, M Pitt, J Curtsinger, X Luo, S Lopez-Verges, LL Lanier, D Weisdorf and JS Miller. (2012). Cytomegalovirus reactivation after allogeneic transplantation promotes a lasting increase in educated NKG2C+ natural killer cells with potent function. Blood 119:2665-74.
39. Foley B, S Cooley, MR Verneris, J Curtsinger, X Luo, EK Waller, DJ Weisdorf and JS Miller. (2011). NK cell education after allogeneic transplantation: dissociation between recovery of cytokine-producing and cytotoxic functions. Blood 118:2784-92.
40. Juelke K, M Killig, A Thiel, J Dong and C Romagnani. (2009). Education of hyporesponsive NK cells by cytokines. Eur J Immunol 39:2548-55.
41. Eissens DN, A Van Der Meer, B Van Cranenbroek, FW Preijers and I Joosten. (2010). Rapamycin and MPA, but not CsA, impair human NK cell cytotoxicity due to differential effects on NK cell phenotype. American Journal of Transplantation 10:1981-90.
42. Wang H, B Grzywacz, D Sukovich, V McCullar, Q Cao, AB Lee, BR Blazar, DN Cornfield, JS Miller and MR Verneris. (2007). The unexpected effect of cyclosporin A on CD56+CD16- and CD56+CD16+ natural killer cell subpopulations. Blood 110:1530-9.
43. Miller JS, KA Alley and P McGlave. (1994). Differentiation of natural killer (NK) cells from human primitive marrow progenitors in a stroma-based long-term culture system: identification of a CD34+7+ NK progenitor. Blood 83:2594-601.
44. Mrozek E, P Anderson and MA Caligiuri. (1996). Role of interleukin-15 in the development of human CD56+ natural killer cells from CD34+ hematopoietic progenitor cells. Blood 87:2632-40.
45. Yoon SR, YS Lee, SH Yang, KH Ahn, JH Lee, JH Lee, DY Kim, YA Kang, M Jeon, M Seol, SG Ryu, JW Chung, I Choi and KH Lee. (2010). Generation of donor natural killer cells from CD34(+) progenitor cells and subsequent infusion after HLA-mismatched allogeneic hematopoietic cell transplantation: a feasibility study. Bone Marrow Transplant 45:1038-46.
46. Shi J, G Tricot, S Szmania, N Rosen, TK Garg, PA Malaviarachchi, A Moreno, B Dupont, KC Hsu, LA Baxter-Lowe, M Cottler-Fox, JD Shaughnessy, Jr., B Barlogie and F van Rhee. (2008). Infusion of haplo-identical killer immunoglobulin-like receptor ligand mismatched NK cells for relapsed myeloma in the setting of autologous stem cell transplantation. Br J Haematol 143:641-53.
47. Choi I, SR Yoon, SY Park, H Kim, SJ Jung, YJ Jang, M Kang, YI Yeom, JL Lee, DY Kim, YS Lee, YA Kang, M Jeon, M Seol, JH Lee, JH Lee, HJ Kim, SC Yun and KH Lee. (2014). Donor-derived natural killer cells infused after human leukocyte antigen-haploidentical hematopoietic cell transplantation: a dose-escalation study. Biol Blood Marrow Transplant 20:696-704.
48. Passweg JR, A Tichelli, S Meyer-Monard, D Heim, M Stern, T Kuhne, G Favre and A Gratwohl. (2004). Purified donor NK-lymphocyte infusion to consolidate engraftment after haploidentical stem cell transplantation. Leukemia 18:1835-8.
49. Koehl U, C Brehm, S Huenecke, SY Zimmermann, S Kloess, M Bremm, E Ullrich, J Soerensen, A Quaiser, S Erben, C Wunram, T Gardlowski, E Auth, T Tonn, C Seidl, S Meyer-Monard, M Stern, J Passweg, T Klingebiel, P Bader, D Schwabe and R Esser. (2013). Clinical grade purification and expansion of NK cell products for an optimized manufacturing protocol. Front Oncol 3:118.
50. McKenna DH, Jr., D Sumstad, N Bostrom, DM Kadidlo, S Fautsch, S McNearney, R Dewaard, PB McGlave, DJ Weisdorf, JE Wagner, J McCullough and JS Miller. (2007). Good manufacturing practices production of natural killer cells for immunotherapy: a six-year single-institution experience. Transfusion 47:520-8.
51. Wilson CL and S Safe. (1998). Mechanisms of ligand-induced aryl hydrocarbon receptor-mediated biochemical and toxic responses. Toxicol Pathol 26:657-71.
52. Denison MS and SR Nagy. (2003). Activation of the aryl hydrocarbon receptor by structurally diverse exogenous and endogenous chemicals. Annu Rev Pharmacol Toxicol 43:309-34.
53. Bessede A, M Gargaro, MT Pallotta, D Matino, G Servillo, C Brunacci, S Bicciato, EM Mazza, A Macchiarulo, C Vacca, R Iannitti, L Tissi, C Volpi, ML Belladonna, C Orabona, R Bianchi, TV Lanz, M Platten, MA Della Fazia, D Piobbico, T Zelante, H Funakoshi, T Nakamura, D Gilot, MS Denison, GJ Guillemin, JB DuHadaway, GC Prendergast, R Metz, M Geffard, L Boon, M Pirro, A Iorio, B Veyret, L Romani, U Grohmann, F Fallarino and P Puccetti. (2014). Aryl hydrocarbon receptor control of a disease tolerance defence pathway. Nature 511:184-90.
54. Casado FL, KP Singh and TA Gasiewicz. (2010). The aryl hydrocarbon receptor: regulation of hematopoiesis and involvement in the progression of blood diseases. Blood Cells Mol Dis 44:199-206.
55. Shertzer HG and AP Senft. (2000). The micronutrient indole-3-carbinol: implications for disease and chemoprevention. Drug Metabol Drug Interact 17:159-88.
56. Lowe MM, JE Mold, B Kanwar, Y Huang, A Louie, MP Pollastri, C Wang, G Patel, DG Franks, J Schlezinger, DH Sherr, AE Silverstone, ME Hahn and JM McCune. (2014). Identification of cinnabarinic acid as a novel endogenous aryl hydrocarbon receptor ligand that drives IL-22 production. PLoS One 9:e87877.
57. Wei YD, H Helleberg, U Rannug and A Rannug. (1998). Rapid and transient induction of CYP1A1 gene expression in human cells by the tryptophan photoproduct 6-formylindolo[3,2-b]carbazole. Chem Biol Interact 110:39-55.
58. Mezrich JD, JH Fechner, X Zhang, BP Johnson, WJ Burlingham and CA Bradfield. (2010). An interaction between kynurenine and the aryl hydrocarbon receptor can generate regulatory T cells. J Immunol 185:3190-8.
59. Punzel M1, Gupta P, Roodell M, Mortari F and Verfaillie CM. (1993). Factor(s) secreted by AFT024 fetal liver cells following stimulation with human cytokines are important for human LTC-IC growth. Leukemia 13:1079-84.
60. Curti A, Ruggeri L, Parisi S, Bontadini A, Dan E, Motta MR, Rizzi S, Trabanelli S, Ocadlikova D, Lecciso M, Giudice V, Fruet F, Urbani E, Papayannidis C, Martinelli G, Bandini G, Bonifazi F, Lewis RE, Cavo M, Velardi A, Lemoli RM. (2016). Larger Size of Donor Alloreactive NK Cell Repertoire Correlates with Better Response to NK Cell Immunotherapy in Elderly Acute Myeloid Leukemia Patients. Clin Cancer Res. Apr 15:22(8): 1914-21.
61. Geller MA, Cooley S, Judson PL, Ghebre R, Carson LF, Argenta PA, Jonson AL, Panoskaltsis-Mortari A, Curtsinger J, McKenna D, Dusenbery K, Bliss R, Downs LS, Miller JS. A phase II study of allogeneic natural killer cell therapy to treat patients with recurrent ovarian and breast cancer. Cytotherapy 13(1):98-107.
62. V. Bachanova, Cooley S, Defor TE, Verneris MR, Zhang B, McKenna DH, Curtsinger J, Panoskaltsis-Mortari A, Lewis D, Hippen K, McGlave P, Weisdorf DJ, Blazar BR, Miller JS. (2014). Clearance of acute myeloid leukemia by haploidentical natural killer cells is improved using IL-2 diphtheria toxin fusion protein. Blood 123(25):3855-63.
63. Sutlu T, Stellan B, Gilljam M, Quezada HC, Nahi H, Gahrton G, Alici E. (2010). Clinical-grade, large-scale, feeder-free expansion of highly active human natural killer cells for adoptive immunotherapy using an automated bioreactor. Cytotherapy. 12(8): 1044-55.
64. Denman CJ, Senyukov VV, Somanchi SS, Phatarpekar PV, Kopp LM, Johnson JL, Singh H, Hurton L, Maiti SN, Huls MH, Champlin RE, Cooper LJ, Lee DA. (2012). Membrane-bound IL-21 promotes sustained ex vivo proliferation of human natural killer cells. PLoS One. 7(1):e30264.
65. Shah N, Martin-Antonio B, Yang H, Ku S, Lee DA, Cooper LJ, Decker WK, Li S, Robinson SN, Sekine T, Parmar S, Gribben J, Wang M, Rezvani K, Yvon E, Najjar A, Burks J, Kaur I, Champlin RE, Bollard CM, Shpall EJ. (2013). Antigen presenting cell-mediated expansion of human umbilical cord blood yields log-scale expansion of natural killer cells with anti-myeloma activity. PLoS One. 8(10):e76781.
66. Roeven MW, Thordardottir S, Kohela A, Maas F, Preijers F, Jansen JH, Blijlevens NM, Cany J, Schaap N, Dolstra H. (2015). The Aryl Hydrocarbon Receptor Antagonist StemRegenin1 Improves In Vitro Generation of Highly Functional Natural Killer Cells from CD34(+) Hematopoietic Stem and Progenitor Cells. Stem Cells Dev. 24(24):2886-2898.
67. Hermanson DL, Bendzick L, Pribyl L, McCullar V, Vogel RI, Miller JS, Geller MA, Kaufman DS. (2014). Induced Pluripotent Stem Cell-Derived Natural Killer Cells for Treatment of Ovarian Cancer. Stem Cells 123(25):3855-63.
68. Cany J, van der Waart AB, Spanholtz J, Tordoir M, Jansen JH, van der Voort R, Schaap NM, Dolstra H. (2015). Combined IL-15 and IL-12 drives the generation of CD34+-derived natural killer cells with superior maturation and alloreactivity potential following adoptive transfer. Oncoimmunology. 4(7):e1017701.
69. Dezell SA, Ahn YO, Spanholtz J, Wang H, Weeres M, Jackson S, Cooley S, Dolstra H, Miller JS, Verneris MR. Natural killer cell differentiation from hematopoietic stem cells: a comparative analysis of heparin- and stromal cell-supported methods. Biol Blood Marrow Transplant. 2012 Apr;18(4):536-45.
70. Ambrosini P, Loiacono F, Conte R, Moretta L, Vitale C, Mingari MC. (2015). IL-1β inhibits ILC3 while favoring NK-cell maturation of umbilical cord blood CD34+ precursors. Eur J Immunol 45:2061-2071.

## Claims

1. A method for the *ex vivo* production of a population of highly functional NK cells from CD34-positive cells, wherein a population of highly functional NK cells is defined as a population wherein at least 10% of the CD56-positive cells are capable of secreting IFN-gamma upon stimulation with K562 target cells and wherein the amount of CD3-positive cells is at most about 0.1%, comprising:
- culturing CD34-positive cells from a single donor in an expansion medium comprising IL-7, SCF, TPO, Flt3L and an aryl hydrocarbon receptor antagonist,
- further culturing the expanded CD34-positive cells in a first differentiation medium Il-7, SCF, Flt3L, IL-15 and an aryl hydrocarbon receptor antagonist,
- culturing in a second differentiation medium comprising IL-7, SCF and IL-15,
wherein at least about 1E+9 highly functional NK cells are produced from a single donor, with the proviso that the expansion medium and the differentiation media do not comprise a glycosaminoglycan and do not comprise G-CSF, GM-CSF or IL-6.

2. A method according to claim 1, wherein the CD34-positive cells are preferably peripheral blood, bone marrow or umbilical cord blood-derived CD34-positive cells.

3. A method according to claim 1 or 2, wherein the aryl hydrocarbon receptor antagonist is one selected from the group consisting of SR1, CH-223191, GNF351, 6,2',4'trimethoxyflavone and CB7993113, preferably the aryl hydrocarbon receptor antagonist is SR1.

4. A method according to claim 3, wherein culturing in the expansion medium is performed for about 7 to 10 days, wherein culturing in the first differentiation medium is performed from about day 7 to 10 to about day 19 to 21, and wherein culturing in the second differentiation medium is performed from about day 19 to 21 for at least about 10 days, preferably for at least about 14 to 21 days or for at least 21 days.

5. A method according to any one of claims 1-4, wherein at least about 1E+9 highly functional and highly pure CD56-positive, Perforin-positive and EOMES-positive NK cells are produced from a single donor.

6. A method according to any one of claims 1-5, wherein the mean overall expansion is at least about two-fold higher than when no aryl hydrocarbon receptor antagonist is used.

7. A population comprising at least 1E+9 highly functional NK cells from a single donor obtainable by a method according to any one of claims 1-6, wherein the amount of CD3-positive cells is at most about 0.1%, and wherein at least 10% of the CD56-positive cells are capable of secreting IFN-gamma upon stimulation with K562 target cells.

8. The population of highly functional NK cells according to claim 7, for use as a medicament.

9. The population of highly functional NK cells according to claim 7 for use in the treatment of cancer.

10. The population of highly functional NK cells for use according to claim 9, wherein treatment of the cancer further comprises allogeneic stem cell transplantation.

## Patentansprüche

1. Verfahren zur Ex-vivo-Produktion einer Population hochfunktioneller NK-Zellen aus CD34-positiven Zellen, wobei eine Population hochfunktioneller NK-Zellen als Population definiert ist, bei der mindestens 10 % der CD56-positiven Zellen in der Lage sind, IFN-gamma bei Stimulation mit K562-Zielzellen abzusondern und wobei die Menge an CD3-positiven Zellen höchstens etwa 0,1 % beträgt, umfassend:
- Kultivieren von CD34-positiven Zellen eines einzigen Spenders in einem Expansionsmedium, das IL-7, SCF, TPO, Flt3L und einen Aryl-Kohlenwasserstoff-Rezeptor-Antagonisten umfasst,
- weiteres Kultivieren der expandierten CD34-positiven Zellen in einem ersten Differenzierungsmedium II-7, SCF, Flt3L, IL-15 und einem Aryl-Kohlenwasserstoff-Rezeptor-Antagonisten,
- Kultivieren in einem zweiten Differenzierungsmedium, das IL-7, SCF und IL-15 umfasst, wobei mindestens etwa 1E+9 hochfunktionelle NK-Zellen von einem einzigen Spender produziert werden, mit der Maßgabe, dass das Expansionsmedium und die Differenzierungsmedien kein Glykosaminoglycan enthalten und kein G-CSF, GM-CSF oder IL-6 enthalten.

2. Verfahren nach Anspruch 1, wobei die CD34-positiven Zellen vorzugsweise aus peripherem Blut, Knochenmark- oder Nabelschnurblut gewonnene CD34-positive Zellen sind.

3. Verfahren nach Anspruch 1 oder 2, wobei der Aryl-Kohlenwasserstoff-Rezeptor-Agonist einer ist, der aus der Gruppe ausgewählt ist, die aus SR1, CH-223191, GNF351, 6,2',4'-Trimethoxyflavon und CB7993113 besteht, wobei der Aryl-Kohlenwasserstoff-Rezeptor-Antagonist vorzugsweise SR1 ist.

4. Verfahren nach Anspruch 3, wobei das Kultivieren in dem Expansionsmedium für etwa 7 bis 10 Tage ausgeführt wird, wobei das Kultivieren in dem ersten Differenzierungsmedium von etwa dem 7. bis 10. Tag bis etwa dem 19. bis 21. Tag ausgeführt wird und wobei das Kultivieren in dem zweiten Differenzierungsmedium von etwa dem 19. bis 21. Tag für mindestens etwa 10 Tage, vorzugsweise für mindestens etwa 14 bis 21 Tage oder für mindestens 21 Tage ausgeführt wird.

5. Verfahren nach einem der Ansprüche 1 - 4, wobei mindestens etwa 1E+9 hochfunktionelle und hochreine CD56-positive, Perforin-positive und EOMES-positive NK-Zellen von einem einzigen Spender produziert werden.

6. Verfahren nach einem der Ansprüche 1 - 5, wobei die mittlere Gesamtexpansion mindestens etwa zweifach höher ist, als wenn kein Aryl-Kohlenwasserstoff-Rezeptor-Antagonist verwendet wird.

7. Population, die mindestens 1E+9 hochfunktionelle NK-Zellen eines einzigen Spenders umfasst, erhältlich durch ein Verfahren nach einem der Ansprüche 1 - 6, wobei die Menge an CD3-positiven Zellen höchstens etwa 0,1 % beträgt und wobei mindestens 10 % der CD56-positiven Zellen in der Lage sind, IFN-gamma bei Stimulation mit K562-Zielzellen abzusondern.

8. Population hochfunktioneller NK-Zellen nach Anspruch 7 zur Verwendung als Medikament.

9. Population hochfunktioneller NK-Zellen nach Anspruch 7 zur Verwendung bei der Krebsbehandlung.

10. Population hochfunktioneller NK-Zellen zur Verwendung nach Anspruch 9, wobei die Krebsbehandlung ferner allogene Stammzelltransplantation umfasst.

## Revendications

1. Procédé de production *ex vivo* d'une population de cellules NK hautement fonctionnelles à partir de cellules CD34 positives, où une population de cellules NK hautement fonctionnelles est définie comme une population où au moins 10 % des cellules CD56 positives sont capables de sécréter de l'IFN-gamma lors d'une stimulation avec des cellules cibles K562 et où la quantité de cellules CD3 positives est au maximum d'environ 0,1 %, comprenant :
- cultiver des cellules CD34 positives provenant d'un seul donneur dans un milieu d'expansion comprenant de l'IL-7, du SCF, de la TPO, du Flt3L et un antagoniste du récepteur des hydrocarbures du groupe aryle,
- cultiver ensuite les cellules CD34 positives expansées dans un premier milieu de différenciation comprenant de l'IL-7, du SCF, du Flt3L, de l'IL-15 et un antagoniste du récepteur des hydrocarbures du groupe aryle,
- cultiver dans un deuxième milieu de différenciation comprenant de l'IL-7, du SCF et de l'IL-15, où au moins environ 1E+9 cellules NK hautement fonctionnelles sont produites à partir d'un seul donneur, à condition que le milieu d'expansion et les milieux de différenciation ne comprennent pas un glycosaminoglycane et ne comprennent pas de G-CSF, de GM-CSF ou d'IL-6.

2. Procédé selon la revendication 1, où les cellules CD34 positives sont de préférence des cellules CD34 positives issues du sang périphérique, de la moelle osseuse ou du sang de cordon ombilical.

3. Procédé selon la revendication 1 ou 2, où l'antagoniste du récepteur des hydrocarbures du groupe aryle est l'un sélectionné dans le groupe consistant en SR1, CH-223191, GNF351, 6,2',4'triméthoxyflavone et CB7993113, de préférence l'antagoniste du récepteur des hydrocarbures du groupe aryle est SR1.

4. Procédé selon la revendication 3, où la culture dans le milieu d'expansion est effectuée pendant environ 7 à 10 jours, où la culture dans le premier milieu de différenciation est effectuée environ du jour 7 au 10 jusqu'à environ du jour 19 au 21, et où la culture dans le deuxième milieu de différenciation est effectuée environ du jour 19 au 21 pendant au moins environ 10 jours, de préférence pendant au moins environ de 14 à 21 jours ou pendant au moins 21 jours.

5. Procédé selon l'une quelconque des revendications 1 à 4, où au moins environ 1E+9 cellules NK hautement fonctionnelles et hautement pures CD56 positives, Perforine positives et EOMES positives sont produites à partir d'un seul donneur.

6. Procédé selon l'une quelconque des revendications 1 à 5, où l'expansion globale moyenne est d'au moins environ deux fois supérieure que lorsqu'aucun antagoniste du récepteur des hydrocarbures du groupe aryle n'est utilisé.

7. Population comprenant au moins 1E+9 cellules NK hautement fonctionnelles provenant d'un seul donneur pouvant être obtenue par un procédé selon l'une quelconque des revendications 1 à 6, où la quantité de cellules CD3 positives est au maximum d'environ 0,1%, et où au moins 10 % des cellules CD56 positives sont capables de sécréter de l'IFN-gamma lors d'une stimulation avec des cellules cibles K562.

8. Population de cellules NK hautement fonctionnelles selon la revendication 7, pour utilisation comme médicament.

9. Population de cellules NK hautement fonctionnelles selon la revendication 7 pour utilisation dans le traitement du cancer.

10. Population de cellules NK hautement fonctionnelles pour utilisation selon la revendication 9, où le traitement du cancer comprend en outre une transplantation allogénique de cellules souches.
